# EUROPÄISCHE PATENTANMELDUNG

(11) **EP 1 595 873 A1**
(43) Veröffentlichungstag der Anmeldung: **16.11.2005**
(21) Anmeldenummer: 04425343.3
(22) Anmeldetag: 13.05.2004
(51) Int. Cl.: C07D 265/28, A61K 31/538, C07C 215/30, A61P 11/00

(54) **Substituierte Cycloalkylderivate zur Behandlung von Atemswegerkrankungen**

(71) Anmelder: Boehringer Ingelheim Pharma GmbH & Co.KG, 55216 Ingelheim am Rhein (DE)
(72) Erfinder: Die Erfindernennung liegt noch nicht vor
(74) Vertreter: Minoja, Fabrizio

(57) **Zusammenfassung**

Die vorliegende Erfindung betrifft Verbindungen der Formel **1** 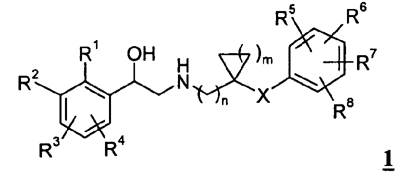 worin die Gruppen R¹, R², R³, R⁴, R⁵, R⁶, R⁷, R⁸, n, m und X die in den Ansprüchen und in der Beschreibung genannten Bedeutungen haben können, sowie deren Verwendung als Arzneimittel, insbesondere zur Behandlung von entzündlichen und obstruktiven Atemwegserkrankungen.

## Beschreibung

Die vorliegende Erfindung betrifft Verbindungen der Formel **1** 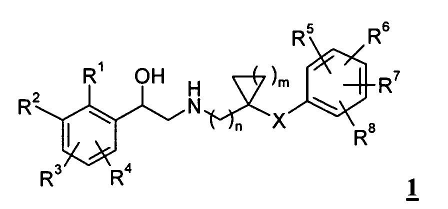 worin die Gruppen R¹, R², R³, R⁴, R⁵, R⁶, R⁷, R⁸, n, m und X die in den Ansprüchen und in der Beschreibung genannten Bedeutungen haben können, sowie deren Verwendung als Arzneimittel, insbesondere zur Behandlung von entzündlichen und obstruktiven Atemwegserkrankungen.

### Hintergrund der Erfindung

Betamimetika (β-adrenerge Substanzen) sind aus dem Stand der Technik bekannt (WO9533724; J.Med.Chem. 1976, 9, 626). Zur medikamentösen Therapie von Erkrankungen ist es häufig wünschenswert, Arzneimittel mit einer längeren Wirkungsdauer bereitzustellen. Hierdurch kann in der Regel gewährleistet werden, dass die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im Übrigen in hohem Maße zum Wohlbefinden des Patienten bei.

Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, dass der Patient sich relativ schnell an die regelmäßige Einnahme des Medikaments zu bestimmten Tageszeiten gewöhnen kann.

Es ist daher Aufgabe der vorliegenden Erfindung, Betamimetika bereitzustellen, die durch eine längere Wirkdauer gekennzeichnet sind und somit zur Herstellung von Arzneimitteln mit längerer Wirksamkeit Verwendung finden können. Es ist insbesondere Aufgabe der vorliegenden Erfindung, Betamimetika bereitzustellen, die aufgrund ihrer langen Wirksamkeit zur Herstellung eines einmal täglich applizierbaren Arzneimittels eingesetzt werden können. Ein weiteres Ziel der vorliegenden Erfindung ist die Bereitstellung von neuen Betamimetika, die aufgrund ihrer langen Wirksamkeit zur Herstellung eines einmal täglich applizierbaren Arzneimittels zur Behandlung von entzündlichen oder obstruktiven Atemwegserkrankungen verwendet werden können. Neben den vorstehend genannten Aufgaben ist es ferner Ziel der vorliegenden Erfindung, solche Betamimetika bereitzustellen, die nicht nur außerordentlich potent, sondern ferner durch ein hohes Maß an Selektivität gegenüber dem β2-Adrenozeptor gekennzeichnet sind.

### Beschreibung der Erfindung

Überraschenderweise wurde gefunden, dass die vorstehend genannten Aufgaben durch Verbindungen der Formel **1** gelöst werden. Dementsprechend betrifft die vorliegende Erfindung Verbindungen der Formel **1** 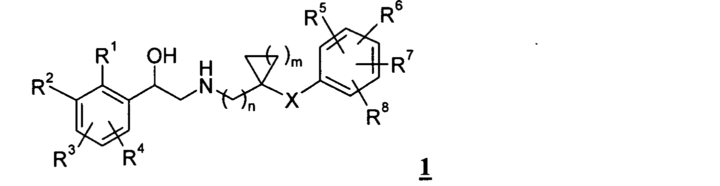 worin
- n: 0 oder 1;
- m: 1, 2, 3 oder 4;
- X: eine Einfachbindung oder eine der zweibindigen Gruppen C₂-C₆-Alkenylen, -O-C₁-C₆-Alkylen, -NH-C₁-C₆-Alkylen, -S-C₁-C₆-Alkylen oder C₁-C₆-Alkylen;
- R¹: Wasserstoff;
- R²: -C₁-C₄-Hydroxyalkyl oder Halogen,
oder
R¹ und R² gemeinsam eine zweibindige Gruppe ausgewählt aus
-O-CH₂-C(O)-NH-, -CH₂-CH₂-C(O)-NH-, -CH=CH-C(O)-NH-,
-NH-CH₂-C(O)-NH-, -S-CH₂-C(O)-NH-, -O-C(O)-NH-, -S-C(O)-NH-,
-NH-C(O)-NH-, und -O-CH₂-SO₂-NH-, in der jeweils ein oder 2 Wasserstoffatome ersetzt sein können durch einen oder zwei Reste ausgewählt aus der Gruppe bestehend aus -C₁-C₄-Alkyl, OH, oder Halogen;
- R³ und R⁴: gleich oder verschieden, ein Rest ausgewählt aus der Gruppe bestehend aus Wasserstoff, OH, Halogen, -C₁-C₆-Alkyl,-C₁-C₆-Haloalkyl,
-C₁-C₆-Hydroxyalkyl, NH₂, NH(-C₁-C₄-Alkyl) und N(-C₁-C₄-Alkyl)₂;
- R⁵, R⁶, R⁷ und R⁸,: gleich oder verschieden, Wasserstoff, OR⁹, -C₁-C₆-Alkyl,
-C₁-C₆-Haloalkyl, -C₁-C₆-Hydroxyalkyl, -C₃-C₆-Cycloalkyl,
-C₃-C₆-Hydroxycycloalkyl, -CN, NO₂, -COR⁹, -COOR⁹, -CONR¹⁰R¹¹, -NR¹⁰R¹¹, -NR¹⁰COR⁹, -NR¹⁰SO₂R¹², -SR¹², -SOR¹², -SO₂R¹², -SO₂NR¹⁰R¹¹ oder Halogen, oder
zwei der Reste R⁵, R⁶, R⁷ und R⁸ bilden, sofern sie am substituierenden Phenylring vicinal ständig sind, gemeinsam eine zweibindige Gruppe ausgewählt aus C₂-C₆-Alkylen, C₂-C₆-Alkenylen und -O-C₁-C₆-alkylen-O- in der jeweils ein oder 2 Wasserstoffatome ersetzt sein können durch einen oder zwei Reste ausgewählt aus der Gruppe bestehend aus -C₁-C₄-Alkyl, -C₁-C₄-Alkoxy, OH, oder Halogen;
- R⁹: Wasserstoff, C₁-C₄-Alkyl, C₆-C₁₀-Aryl oder C₆-C₁₀-Aryl-C₁-C₄-alkylen;
- R¹⁰ und R¹¹: gleich oder verschieden, Wasserstoff, C₁-C₄-Alkyl, C₆-C₁₀-Aryl oder C₆-C₁₀-Aryl-C₁-C₄-alkylen;
- R¹²: C₁-C₄-Alkyl, C₆-C₁₀-Aryl oder C₆-C₁₀-Aryl-C₁-C₄-alkylen,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Bevorzugt sind Verbindungen der allgemeinen Formel **1**,
worin
- n: 0 oder 1;
- m: 1, 2, 3 oder 4;
- X: eine Einfachbindung oder eine der zweibindigen Gruppen C₂-C₄-Alkenylen,
-O-C₁-C₄-Alkylen, -NH-C₁-C₄-Alkylen, -S-C₁-C₄-Alkylen oder C₁-C₄-Alkylen;
- R¹: Wasserstoff;
- R²: -CH₂-CH₂-OH, -CH₂-OH, Fluor, Chlor oder Brom
oder
R¹ und R² gemeinsam eine zweibindige Gruppe ausgewählt aus -O-CH₂-C(O)-NH-, -CH₂-CH₂-C(O)-NH-, -CH=CH-C(O)-NH-, -NH-CH₂-C(O)-NH-, -O-C(O)-NH-, -NH-C(O)-NH- und -O-CH₂-SO₂-NH-, in der jeweils ein oder 2 Wasserstoffatome ersetzt sein können durch einen oder zwei Reste ausgewählt aus der Gruppe bestehend aus Methyl, OH, Fluor, Chlor, oder Brom;
- R³ und R⁴: gleich oder verschieden, ein Rest ausgewählt aus der Gruppe bestehend aus Wasserstoff, OH, Halogen, -C₁-C₄-Alkyl, -C₁-C₄-Haloalkyl,
-C₁-C₄-Hydroxyalkyl, NH₂, NH(-C₁-C₄-Alkyl) und N(-C₁-C₄-Alkyl)₂;
- R⁵, R⁶, R⁷ und R⁸,: gleich oder verschieden, Wasserstoff, OR⁹, -C₁-C₄-Alkyl, -C₁-C₄-Haloalkyl, -C₁-C₄-Hydroxyalkyl, -C₃-C₆-Cycloalkyl, -C₃-C₆-Hydroxycycloalkyl, -CN, NO₂, -COR⁹, -COOR⁹, -CONR¹⁰R¹¹, -NR¹⁰R¹¹, -NR¹⁰COR⁹, -NR¹⁰SO₂R¹², -SR¹², -SOR¹², -SO₂R¹², -SO₂NR¹⁰R¹¹, Fluor, Chlor oder Brom, oder
zwei der Reste R⁵, R⁶, R⁷ und R⁸ bilden, sofern sie am substituierenden Phenylring vicinal ständig sind, gemeinsam eine zweibindige Gruppe ausgewählt aus C₂-C₄-Alkylen, C₂-C₄-Alkenylen und -O-C₁-C₄-Alkylen-O- in der jeweils ein oder 2 Wasserstoffatome ersetzt sein können durch einen oder zwei Reste ausgewählt aus der Gruppe bestehend aus Methyl, Methoxy, OH, Fluor, Chlor oder Brom;
- R⁹: Wasserstoff, Methyl, Ethyl, Phenyl, Naphthyl, Benzyl, Naphthylmethyl oder 2- Phenylethyl;
- R¹⁰ und R¹¹: gleich oder verschieden, Wasserstoff, Methyl, Ethyl, Phenyl, Naphthyl, Naphthylmethyl, Benzyl oder 2-Phenylethyl;
- R¹²: Methyl, Ethyl, Phenyl, Naphthyl, Naphthylmethyl, Benzyl oder 2-Phenylethyl;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind Verbindungen der allgemeinen Formel **1**,
worin
- n: 0 oder 1;
- m: 1, 2, 3 oder 4;
- X: eine Einfachbindung oder eine der zweibindigen Gruppen -CH₂-, -CH₂-CH₂-, -CH₂-CH₂-CH₂-, -CH=CH-, -CH₂-CH=CH-, -CH₂-O-, -CH₂-CH₂-O-, -CH₂-NH₂-, -CH₂-CH₂-NH₂-;
- R¹: Wasserstoff;
- R²: -CH₂-CH₂-OH, -CH₂-OH, Fluor, Chlor oder Brom
oder
R¹ und R² gemeinsam eine zweibindige Gruppe ausgewählt aus
-O-CH₂-C(O)-NH-, -CH₂-CH₂-C(O)-NH-, -CH=CH-C(O)-NH-, oder
-O-C(O)-NH-, in der jeweils ein oder 2 Wasserstoffatome ersetzt sein können durch einen oder zwei Reste ausgewählt aus der Gruppe bestehend aus Methyl, OH,
Fluor, Chlor, oder Brom, bevorzugt Methyl;
- R³ und R⁴: gleich oder verschieden, ein Rest ausgewählt aus der Gruppe bestehend aus Wasserstoff, OH, Fluor, Chlor, Brom, Methyl, Ethyl, -CH₂F, -CHF₂, -CF₃, -CH₂-CH₂-OH, -CH₂-OH, NH₂, NH(Methyl) und N(Methyl)₂;
- R⁵, R⁶, R⁷ und R⁸,: gleich oder verschieden, Wasserstoff, OH, Methyl, Ethyl, Propyl, Butyl, -CH₂F, -CHF₂, -CF₃, -CH₂-CH₂F, -CH₂-CHF₂, -CH₂-CF₃, -CH₂-CH₂-OH, -CH₂-OH, Cyclpropyl, Cyclobutyl, Cyclopentyl, HO-Cyclpropyl, HO-Cyclobutyl, HO-Cyclopentyl, -CN, NO₂, -COPhenyl, -COOMethyl, -COOEthyl, -CONH₂, -CONHMethyl, -CONHPhenyl, -CONHBenzyl, -CON(Methyl)₂, NH₂, NH(Methyl), N(Methyl)₂, -NHCOMethyl, -NHCOPhenyl, -NHSO₂Methyl, -NHSO₂Phenyl, -NHSO₂-Phenyl-CH₃, -SO₂Methyl, -SO₂-Phenyl, -SO₂-Phenyl-CH₃, -SO₂NH₂, Fluor, Chlor oder Brom, oder
zwei der Reste R⁵, R⁶, R⁷ und R⁸ bilden, sofern sie am substituierenden Phenylring vicinal ständig sind, gemeinsam eine zweibindige Gruppe ausgewählt aus -CH₂- CH₂-CH₂-, -CH₂-CH₂-CH₂-CH₂-, -CH₂-CH=CH-,
-CH=CH-CH=CH-, -O-CH₂-O- und -O-CH₂-CH₂-O- in der jeweils ein oder 2 Wasserstoffatome ersetzt sein können durch einen oder zwei Reste ausgewählt aus der Gruppe bestehend aus Methyl, Methoxy, OH oder Fluor,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind Verbindungen der Formel **1**, worin
- X: eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-, bedeutet
und worin die Gruppen R¹, R², R³, R⁴, R⁵, R⁶, R⁷, R⁸, n und m vorstehend genannte Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind ferner Verbindungen der Formel **1**, worin
- R¹: Wasserstoff;
- R²: Methyl, Ethyl, CHF₂, CH₂F, CF₃, -CH₂-CH₂-OH, -CH₂-OH, Fluor, Chlor oder Brom, oder
R¹ und R² gemeinsam eine zweibindige Gruppe ausgewählt aus -O-CH₂-C(O)-NH-, -CH₂-CH₂-C(O)-NH-, -CH=CH-C(O)-NH- und -CH=CH-CH=CH-, bedeuten,
und worin die Gruppen R³, R⁴, R⁵, R⁶, R⁷, R⁸, n, m und X vorstehend genannte Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind ferner Verbindungen der Formel **1**, worin
- R³ und R⁴: gleich oder verschieden, ein Rest ausgewählt aus der Gruppe bestehend aus Wasserstoff, OH, Fluor, Chlor, Brom, Methyl, Ethyl, CHF₂, CH₂F, CF₃, -CH₂-CH₂-OH, -CH₂-OH, NH₂, NHMethyl, NHEthyl, N(Methyl)₂ und N(Ethyl)₂, bevorzugt ausgewählt aus der Gruppe bestehend aus Wasserstoff, OH, Fluor, CH₂F, CF₃ und NH₂, bedeuten,
und worin die Gruppen R¹, R², R⁵, R⁶, R⁷, R⁸, n, m und X vorstehend genannte Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind ferner Verbindungen der Formel **1**, worin
- R⁵, R⁶, R⁷ und R⁸,: gleich oder verschieden, Wasserstoff, OH, Methyl, Ethyl, Propyl, Butyl, CHF₂, CH₂F, CF₃, -CH₂-CH₂-OH, -CH₂-OH, Methyloxy, Ethyloxy, Propyloxy, Butyloxy, Cyclopropyl, Hydroxycyclopropyl, NH₂, NHMethyl, N(Methyl)₂, Fluor, Chlor oder Brom, oder
zwei der Reste R⁵, R⁶, R⁷ und R⁸ gemeinsam, sofern sie am substituierten Phenylring vicinal ständig sind, die zweibindige Gruppe -CH=CH-CH=CH- in der jeweils ein oder 2 Wasserstoffatome ersetzt sein können durch einen oder zwei Reste ausgewählt aus der Gruppe bestehend aus Methyl,

Methoxy, OH, Fluor, Chlor oder Brom, bedeuten,
und worin die Gruppen R¹, R², R³, R⁴ n, m und X vorstehend genannte Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind ferner Verbindungen der Formel **1**, worin
- R⁵, R⁶, R⁷ und R⁸,: gleich oder verschieden, Wasserstoff, OH, Methyl, Ethyl, Propyl, Butyl, CHF₂, CH₂F, CF₃, Methyloxy, Ethyloxy, Propyloxy, Butyloxy, Cyclopropyl, Hydroxycyclopropyl, NH₂, Fluor, Chlor oder Brom,
bedeuten und worin die Gruppen R¹, R², R³, R⁴ n, m und X vorstehend genannte Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind ferner Verbindungen der Formel **1**, worin
- R⁵, R⁶, R⁷ und R⁸,: gleich oder verschieden, Wasserstoff, OH, Methyl, CF₃, Methyloxy, Ethyloxy, Propyloxy, Butyloxy, Hydroxycyclopropyl, NH₂, Fluor oder Chlor,
bedeuten und worin die Gruppen R¹, R², R³, R⁴ n, m und X vorstehend genannte Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind ferner Verbindungen der Formel **1**, worin
- R⁵ und R⁸: Wasserstoff;
- R⁶ und R⁷: gemeinsam, sofern sie am substituierenden Phenylring vicinal ständig sind, die zweibindige Gruppe -CH=CH-CH=CH- bedeuten,
und worin die Gruppen R¹, R², R³, R⁴ n, m und X vorstehend genannte Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind ferner Verbindungen der Formel **1**, worin
- n: 0;
- m: 2 bedeuten,
und worin die Gruppen R¹, R², R³, R⁴, R⁵, R⁶, R⁷, R⁸ und X vorstehend genannte Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind ferner Verbindungen der Formel **1**, worin
- n: 0;
- m: 3 bedeuten,
und worin die Gruppen R¹, R², R³, R⁴, R⁵, R⁶, R⁷, R⁸ und X vorstehend genannte Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Ein bevorzugter Aspekt der vorliegenden Erfindung betrifft Verbindungen der allgemeinen Formel **1**, worin
- n: 0 oder 1, bevorzugt 0;
- m: 1, 2, 3 oder 4, bevorzugt 1;
- X: eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-,
- R¹: Wasserstoff und
- R²: -CH₂-OH,
oder
R¹ und R² gemeinsam eine zweibindige Gruppe ausgewählt aus -O-CH₂-C(O)-NH-, -CH₂-CH₂-C(O)-NH-, -CH=CH-C(O)-NH-, -NH-CH₂-C(O)-NH-, -S-CH₂-C(O)-NH-, -O-C(O)-NH-, -NH-C(O)-NH-, und -O- CH₂-SO₂-NH;
- R³: Wasserstoff;
- R⁴: OH,
bedeuten und worin die Gruppen R⁵, R⁶, R⁷ und R⁸ vorstehend genannten Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Bevorzugt sind Verbindungen der allgemeinen Formel **1**,
worin
- R⁵, R⁶, R⁷ und R⁸,: gleich oder verschieden, Wasserstoff, OR⁹, -C₁-C₄-Alkyl, -C₁-C₄-Haloalkyl, -C₁-C₄-Hydroxyalkyl, -C₃-C₆-Cycloalkyl, -C₃-C₆-Hydroxycycloalkyl, -CN, NO₂, -COR⁹, -COOR⁹, -CONR¹⁰R¹¹, -NR¹⁰R¹¹, -NR¹⁰COR⁹, -NR¹⁰SO₂R¹², -SR¹², -SOR¹², -SO₂R¹², -SO₂NR¹⁰R¹¹, Fluor, Chlor oder Brom, oder
zwei der Reste R⁵, R⁶, R⁷ und R⁸ bilden, sofern sie am substituierenden Phenylring vicinal ständig sind, gemeinsam eine zweibindige Gruppe ausgewählt aus C₂-C₄-Alkylen, C₂-C₄-Alkenylen und -O-C₁-C₄-Alkylen-O- in der jeweils ein oder 2 Wasserstoffatome ersetzt sein können durch einen oder zwei Reste ausgewählt aus der Gruppe bestehend aus Methyl, Methoxy, OH, Fluor, Chlor oder Brom;
- R⁹: Wasserstoff, Methyl, Ethyl, Phenyl, Naphthyl, Benzyl, Naphthylmethyl oder 2- Phenylethyl;
- R¹⁰ und R¹¹: gleich oder verschieden, Wasserstoff, Methyl, Ethyl, Phenyl, Naphthyl, Naphthylmethyl, Benzyl oder 2-Phenylethyl;
- R¹²: Methyl, Ethyl, Phenyl, Naphthyl, Naphthylmethyl, Benzyl oder 2-Phenylethyl,
bedeuten, und worin die Gruppen R¹, R², R³, R⁴, n, m und X vorstehend genannte Beutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Eine bevorzugte Gruppe erfindungsgemäßer Verbindungen, sind Verbindungen der allgemeinen Formel **1** in denen die Gruppen R¹ und R² gemeinsam die zweibindige Gruppe -O-CH₂-C(O)-NH- bilden und die Gruppen R³, R⁴, R⁵, R⁶, R⁷ R⁸, n, m und X die vorstehend genannten Bedeutungen haben können.

Bevorzugte Regioisomere dieser Gruppe sind durch die allgemeine Formel **1a** 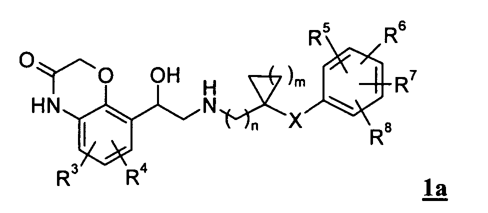 darstellbar.

Ein bevorzugter Aspekt der vorliegenden Erfindung betrifft Verbindungen der allgemeinen Formel **1a**, worin
- n: 0 oder 1, bevorzugt 0;
- m: 1, 2, 3 oder 4, bevorzugt 1;
- X: eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-,
- R³: Wasserstoff;
- R⁴: OH;
- R⁵, R⁶, R⁷ und R⁸,: gleich oder verschieden, Wasserstoff, OR⁹, -C₁-C₄-Alkyl, -C₁-C₄-Haloalkyl, -C₁-C₄-Hydroxyalkyl, -C₃-C₆-Cycloalkyl, -C₃-C₆-Hydroxycycloalkyl, -CN, NO₂, -COR⁹, -COOR⁹, -CONR¹⁰R¹¹, -NR¹⁰R¹¹, -NR¹⁰COR⁹, -NR¹⁰SO₂R¹², -SR¹², -SOR¹², -SO₂R¹², -SO₂NR¹⁰R¹¹, Fluor, Chlor oder Brom, oder
zwei der Reste R⁵, R⁶, R⁷ und R⁸ bilden, sofern sie am substituierenden Phenylring vicinal ständig sind, gemeinsam eine zweibindige Gruppe ausgewählt aus C₂-C₄-Alkylen, C₂-C₄-Alkenylen und -O-C₁-C₄-Alkylen-O- in der jeweils ein oder 2 Wasserstoffatome ersetzt sein können durch einen oder zwei Reste ausgewählt aus der Gruppe bestehend aus Methyl, Methoxy, OH, Fluor, Chlor oder Brom;
- R⁹: Wasserstoff, Methyl, Ethyl, Phenyl, Naphthyl, Benzyl, Naphthylmethyl oder 2- Phenylethyl;
- R¹⁰ und R¹¹: gleich oder verschieden, Wasserstoff, Methyl, Ethyl, Phenyl, Naphthyl, Naphthylmethyl, Benzyl oder 2-Phenylethyl;
- R¹²: Methyl, Ethyl, Phenyl, Naphthyl, Naphthylmethyl, Benzyl oder 2-Phenylethyl,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Bevorzugt sind Verbindungen der allgemeinen Formel **1a**, worin
- n: 0 oder 1, bevorzugt 0;
- m: 1, 2, 3 oder 4, bevorzugt 1;
- X: eine Einfachbindung oder eine der zweibindigen Gruppen -CH₂-, -CH₂-CH₂-, -CH₂-CH₂-CH₂-, -CH=CH-, -CH₂-CH=CH-, -CH₂-O-, -CH₂-CH₂-O-, -CH₂-NH₂-, -CH₂-CH₂-NH₂-;
- R³: Wasserstoff;
- R⁴: Wasserstoff, Fluor, Methyl, OH oder CF₃, bevorzugt OH;
- R⁵ und: R⁸ gleich oder verschieden, Wasserstoff, Methyl, Methyloxy oder Fluor;
- R⁶ und R⁷: gleich oder verschieden, Wasserstoff, OH, Methyl, Ethyl, CF₃, Methyloxy, Ethyloxy, Propyloxy, Butyloxy, Cyclopropyl, Hydroxycyclopropyl, NH₂ oder Fluor,
oder
R⁶ und R⁷, sofern sie am substituierenden Phenylring vicinal ständig sind, gemeinsam die zweibindige Gruppe -CH=CH-CH=CH- in der jeweils ein oder 2 Wasserstoffatome ersetzt sein können durch einen oder zwei Reste ausgewählt aus der Gruppe bestehend aus Methyl, Methoxy, OH, Fluor, Chlor und Brom,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Bevorzugt sind Verbindungen der allgemeinen Formel **1a**, worin
- n: 0 oder 1, bevorzugt 0;
- m: 1, 2, 3 oder 4, bevorzugt 1;
- X: eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-,
- R³: Wasserstoff;
- R⁴: OH;
- R⁵ und R⁸: gleich oder verschieden, Wasserstoff, Methyl, Methyloxy oder Fluor;
- R⁶ und R⁷: gleich oder verschieden, Wasserstoff, OH, Methyl, CF₃, Methyloxy, Ethyloxy, Propyloxy, Butyloxy, Hydroxycyclopropyl, NH₂ oder Fluor, oder
- R⁶ und R⁷,: sofern sie am substituierenden Phenylring vicinal ständig sind, gemeinsam die zweibindige Gruppe -CH=CH-CH=CH-,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind Verbindungen der allgemeinen Formel **1a**, worin
- R⁴: OH;
- R⁵, R⁶ und R⁸: gleich oder verschieden, Wasserstoff oder Methyl, bevorzugt Wasserstoff;
- R⁷: Wasserstoff, OH, Methyl, CF₃, Methyloxy, Ethyloxy, Propyloxy oder Butyloxy, bevorzugt OH, Methyloxy, Ethyloxy, Propyloxy oder Butyloxy, bedeuten, und n, m, X und R³ die vorstehend genannten Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.
Eine bevorzugte Gruppe erfindungsgemäßer Verbindungen, sind Verbindungen der allgemeinen Formel **1** in denen die Gruppen R¹ und R² gemeinsam die zweibindige Gruppe -CH=CH-C(O)-NH- bilden und die Gruppen R³, R⁴, R⁵, R⁶, R⁷, R⁸, n, m und X die vorstehend genannten Bedeutungen haben können.

Bevorzugte Regioisomere dieser Gruppe sind durch die allgemeine Formel **1b** 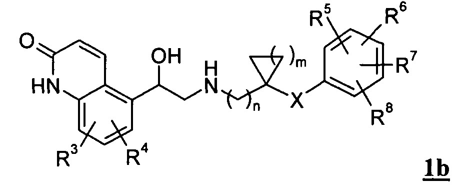 darstellbar.

Ein bevorzugter Aspekt der vorliegenden Erfindung betrifft Verbindungen der allgemeinen Formel **1b**, worin
- n: 0 oder 1, bevorzugt 0;
- m: 1, 2, 3 oder 4, bevorzugt 1;
- X: eine Einfachbindung oder eine der zweibindigen Gruppen -CH₂-, -CH₂-CH₂-, -CH₂-CH₂-CH₂-, -CH=CH-, -CH₂-CH=CH-, -CH₂-O-, -CH₂-CH₂-O-, -CH₂-NH₂-, -CH₂-CH₂-NH₂-;
- R³: Wasserstoff;
- R⁴: Wasserstoff, Fluor, Methyl, OH oder CF₃, bevorzugt OH;
- R⁵ und R⁸: gleich oder verschieden, Wasserstoff, Methyl, Methyloxy oder Fluor;
- R⁶ und R⁷: gleich oder verschieden, Wasserstoff, OH, Methyl, Ethyl, CF₃, Methyloxy, Ethyloxy, Propyloxy, Butyloxy, Cyclopropyl, Hydroxycyclopropyl, NH₂ oder Fluor,
oder
R⁶ und R⁷, sofern sie am substituierenden Phenylring vicinal ständig sind, gemeinsam die zweibindige Gruppe -CH=CH-CH=CH- in der jeweils ein oder 2 Wasserstoffatome ersetzt sein können durch einen oder zwei Reste ausgewählt aus der Gruppe bestehend aus Methyl, Methoxy, OH, Fluor, Chlor und Brom,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Bevorzugt sind Verbindungen der allgemeinen Formel **1b**, worin
- n: 0 oder 1, bevorzugt 0;
- m: X 1, 2, 3 oder 4, bevorzugt 1; eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-,
- R³: Wasserstoff;
- R⁴: OH;
- R⁵ und R⁸: gleich oder verschieden, Wasserstoff, Methyl, Methyloxy oder Fluor;
- R⁶ und R⁷: gleich oder verschieden, Wasserstoff, OH, Methyl, CF₃, Methyloxy, Ethyloxy, Propyloxy, Butyloxy, Hydroxycyclopropyl, NH₂ oder Fluor,
oder
R⁶ und R⁷, sofern sie am substituierenden Phenylring vicinal ständig sind, gemeinsam die zweibindige Gruppe -CH=CH-CH=CH-,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind Verbindungen der allgemeinen Formel **1b**, worin
- n: 0 oder 1, bevorzugt 0;
- m: 1, 2, 3 oder 4, bevorzugt 1;
- X: die zweibindige Gruppe -CH₂-;
- R³: Wasserstoff;
- R⁴: OH;
- R⁵, R⁶ und R⁸: gleich oder verschieden, Wasserstoff oder Methyl, bevorzugt Wasserstoff;
- R⁷: Wasserstoff, OH, Methyl, CF₃, Methyloxy oder Ethyloxy, bevorzugt OH, CF₃ oder Methyloxy,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Eine bevorzugte Gruppe erfindungsgemäßer Verbindungen, sind Verbindungen der allgemeinen Formel **1** in denen R¹ Wasserstoff, R² Chlor, R³ NH₂ und R⁴ Chlor bedeuten und die Gruppen R⁵, R⁶, R⁷, R⁸, n, m und X die vorstehend genannten Bedeutungen haben können.. Bevorzugte Regioisomere dieser Gruppe sind durch die allgemeine Formel **1c** 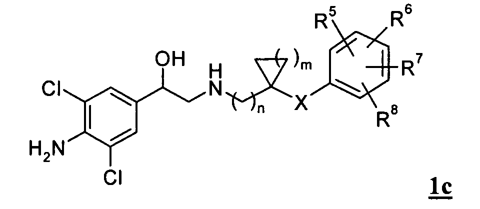 darstellbar.

Ein bevorzugter Aspekt der vorliegenden Erfindung betrifft Verbindungen der allgemeinen Formel **1c**, worin
- n: 0 oder 1, bevorzugt 0;
- m: 1, 2, 3 oder 4, bevorzugt 1;
- X: eine Einfachbindung oder eine der zweibindigen Gruppen -CH₂-, -CH₂-CH₂-, -CH₂-CH₂-CH₂-, -CH=CH-, -CH₂-CH=CH-, -CH₂-O-, -CH₂-CH₂-O-, -CH₂-NH₂-, -CH₂-CH₂-NH₂-;
- R⁵ und R⁸: gleich oder verschieden, Wasserstoff, Methyl, Methyloxy oder Fluor;

- R⁶ und R⁷: gleich oder verschieden, Wasserstoff, OH, Methyl, Ethyl, CF₃, Methyloxy, Ethyloxy, Propyloxy, Butyloxy, Cyclopropyl, Hydroxycyclopropyl, NH₂ oder Fluor,
oder
R⁶ und R⁷, sofern sie am substituierenden Phenylring vicinal ständig sind, gemeinsam die zweibindige Gruppe -CH=CH-CH=CH- in der jeweils ein oder 2 Wasserstoffatome ersetzt sein können durch einen oder zwei Reste ausgewählt aus der Gruppe bestehend aus Methyl, Methoxy, OH, Fluor, Chlor und Brom,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Bevorzugt sind Verbindungen der allgemeinen Formel **1c**, worin
- n: 0 oder 1, bevorzugt 0;
- m: 1, 2, 3 oder 4, bevorzugt 1;
- X: eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-,
- R⁵ und R⁸: Wasserstoff;
- R⁶ und R⁷: gleich oder verschieden, Wasserstoff, OH, Methyl, CF₃, Methyloxy oder Ethyloxy,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind Verbindungen der allgemeinen Formel **1c**, worin
- n: 0 oder 1, bevorzugt 0;
- m: 1, 2, 3 oder 4, bevorzugt 1;
- X: die zweibindige Gruppe -CH₂-;
- R⁵, R⁶ und R⁸: gleich oder verschieden, Wasserstoff oder Methyl, bevorzugt Wasserstoff;
- R⁷: Wasserstoff, OH, Methyloxy oder Ethyloxy,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Eine bevorzugte Gruppe erfindungsgemäßer Verbindungen, sind Verbindungen der allgemeinen Formel **1** in denen R¹ Wasserstoff, R² Hydroxymethyl und R³ OH bedeuten und die Gruppen R⁴, R⁵, R⁶, R⁷, R⁸, n, m und X die vorstehend genannten Bedeutungen haben können.

Bevorzugte Regioisomere dieser Gruppe sind durch die allgemeine Formel **1d** 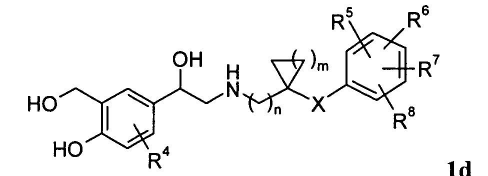 darstellbar.

Ein bevorzugter Aspekt der vorliegenden Erfindung betrifft Verbindungen der allgemeinen Formel **1d**, worin
- n: 0 oder 1, bevorzugt 0;
- m: 1, 2, 3 oder 4, bevorzugt 1;
- X: eine Einfachbindung oder eine der zweibindigen Gruppen -CH₂-, -CH₂-CH₂-, -CH₂-CH₂-CH₂-, -CH=CH-, -CH₂-CH=CH-, -CH₂-O-, -CH₂-CH₂-O-, -CH₂-NH₂-, -CH₂-CH₂-NH₂-;
- R⁴: Wasserstoff, Fluor, Methyl, OH oder CF₃, bevorzugt Wasserstoff;
- R⁵ und R⁸: gleich oder verschieden, Wasserstoff, Methyl, Methyloxy oder Fluor;
- R⁶ und R⁷: gleich oder verschieden, Wasserstoff, OH, Methyl, Ethyl, CF₃, Methyloxy, Ethyloxy, Propyloxy, Butyloxy, Cyclopropyl, Hydroxycyclopropyl, NH₂ oder Fluor,
oder
R⁶ und R⁷, sofern sie am substituierenden Phenylring vicinal ständig sind, gemeinsam die zweibindige Gruppe -CH=CH-CH=CH- in der jeweils ein oder 2 Wasserstoffatome ersetzt sein können durch einen oder zwei Reste ausgewählt aus der Gruppe bestehend aus Methyl, Methoxy, OH, Fluor, Chlor und Brom,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Bevorzugt sind Verbindungen der allgemeinen Formel **1d**, worin
- n: 0 oder 1, bevorzugt 0;
- m: 1, 2, 3 oder 4, bevorzugt 1;
- X: eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-,
- R⁴: Wasserstoff;
- R⁵ und R⁸: gleich oder verschieden, Wasserstoff, Methyl, Methyloxy oder Fluor, bevorzugt Wasserstoff;
- R⁶ und R⁷: gleich oder verschieden, Wasserstoff, OH, Methyl, CF₃, Methyloxy, Ethyloxy, Propyloxy, Butyloxy, NH₂ oder Fluor,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugt sind Verbindungen der allgemeinen Formel **1d**, worin
- n: 0 oder 1, bevorzugt 0;
- m: 1, 2, 3 oder 4, bevorzugt 1;
- X: die zweibindige Gruppe -CH₂-;
- R⁴: Wasserstoff;
- R⁵, R⁶ und R⁸: gleich oder verschieden, Wasserstoff oder Methyl, bevorzugt Wasserstoff;
- R⁷: Wasserstoff, OH, Methyl, CF₃, Methyloxy oder Ethyloxy, bevorzugt OH, CF₃ oder Methyloxy,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Besonders bevorzugte Verbindungen der Formel **1** sind ausgewählt aus der Gruppe bestehend aus:
- 5-Hydroxy-8-{1-hydroxy-2-[1-(4-methoxy-benzyl)-cyclopropylamino]-ethyl}-4H-benzo[1,4]oxazin-3-on ;
- 8-[2-(1-Benzyl-cyclopropylamino)-1-hydroxy-ethyl]-5-hydroxy-4H-benzo[1,4]oxazin-3-on ;
- 8-{2-[1-(2,6-Dimethyl-benzyl)-cyclopropylamino]-1-hydroxy-ethyl}-5-hydroxy-4H-benzo[1,4]oxazin-3-on;
- 8-{2-[1-(3,4-Dimethyl-benzyl)-cyclopropylamino]-1-hydroxy-ethyl}-5-hydroxy-4H-benzo[1,4]oxazin-3-on ;
- 5-Hydroxy-8-{1-hydroxy-2-[1-(3-methyl-benzyl)-cyclopropylamino]-ethyl}-4H-benzo[1,4]oxazin-3-on;
- 5-Hydroxy-8-{1-hydroxy-2-[1-(2-trifluormethyl-benzyl)-cyclopropylamino]-ethyl}-4H-benzo[1,4]oxazin-3-on ;
- 5-Hydroxy-8-(1-hydroxy-2-{1-[4-(1-hydroxy-cyclopropyl)-benzyl]-cyclopropylamino}-ethyl)-4H-benzo[1,4]oxazin-3-on;
- 8-{2-[1-(3,5-Difluor-benzyl)-cyclopropylamino]-1-hydroxy-ethyl}-5-hydroxy-4H-benzo[1,4]oxazin-3-on ;
- 5-Hydroxy-8-[1-hydroxy-2-(1-phenoxymethyl-cyclopropylamino)-ethyl]-4H-benzo[1,4]oxazin-3-on;
- 5-Hydroxy-8-{1-hydroxy-2-[1-(4-trifluormethyl-benzyl)-cyclopropylamino]-ethyl}-4H-benzo[1,4]oxazin-3-on;
- 5-Hydroxy-8-{1-hydroxy-2-[1-(2-methoxy-benzyl)-cyclopropylamino]-ethyl}-4H-benzo[1,4]oxazin-3-on;
- 5-Hydroxy-8-{1-hydroxy-2-[1-(2-methyl-benzyl)-cyclopropylamino]-ethyl}-4H-benzo[1,4]oxazin-3-on;
- 8-{2-[1-(2,6-Dimethyl-phenoxymethyl)-cyclopropylamino]-1-hydroxy-ethyl}-5-hydroxy-4H-benzo[1,4]oxazin-3-on;
- 5-Hydroxy-8-[1-hydroxy-2-(1-naphtalen-2-ylmetyhl-cyclopropylamino)-ethyl]-4H-benzo[1,4]oxazin-3-on;
- 8- {2-[1-(2,4-Dimethyl-benzyl)-cyclopropylamino]-1-hydroxy-ethyl}-5-hydroxy-4H-benzo[1,4]oxazin-3-on;
- 5-Hydroxy-8-{1-hydroxy-2-[1-(2,3,5,6-tetramethyl-benzyl)-cyclopropylamino]-ethyl}-4H-benzo[1,4]oxazin-3-on;
- 5-Hydroxy-8-(1-hydroxy-2-{1-[2-(4-methoxy-phenyl)-ethyl]-cyclopropylamino}-ethyl)-4H-benzo[1,4]oxazin-3-on;
- 8-{2-[1-(2,6-Difluor-benzyl)-cyclopropylamino]-1-hydroxy-ethyl}-5-hydroxy-4H-benzo[1,4]oxazin-3-on;
- 8-{2-[1-(4-Amino-benzyl)-cyclopropylamino]-1-hydroxy-ethyl}-5-hydroxy-4H-benzo[1,4]oxazin-3-on ;
- 5-Hydroxy-8-{1-hydroxy-2-[1-(4-hydroxy-benzyl)-cyclopropylamino]-ethyl}-4H-benzo[1,4]oxazin-3-on;
- 8-{2-[1-(3,4-Dimethoxy-benzyl)-cyclopropylamino]-1-hydroxy-ethyl}-5-hydroxy-4H-benzo[1,4]oxazin-3-on;
- 8-{2-[1-(4-Ethoxy-benzyl)-cyclopropylamino]-1-hydroxy-ethyl}-5-hydroxy-4H-benzo [1,4]oxazin-3-on;
- 8-{2-[1-(4-Butoxy-benzyl)-cyclopropylamino]-1-hydroxy-ethyl}-5-hydroxy-4H-benzo[1,4]oxazin-3-on;
- 5-Hydroxy-8-{1-hydroxy-2-[1-(2,4,6-trimethyl-benzyl)-cyclopropylamino]-ethyl}-4H-benzo[1,4]oxazin-3-on;
- 5-Hydroxy-8-{1-hydroxy-2-[(1-phenyl-cyclopropylmethyl)-amino]-ethyl}-4H-benzo[1,4]oxazin-3-on;
- 5-Hydroxy-8-{1-hydroxy-2-[1-(2-naphthalen-2-yl-ethyl)-cyclopropylamino]-ethyl}-4H-benzo[1,4]oxazin-3-on;
- 2-Hydroxymethyl-4-{1-hydroxy-2-[(1-phenyl-cyclopropylmethyl)-amino]-ethyl}-phenol;
- 4-{1-Hydroxy-2-[1-(4-methoxy-benzyl)-cyclopropylamino]-ethyl}-2-hydroxymethyl-phenol;
- 4-[2-(1-Benzyl-cyclopropylamino)-1-hydroxy-ethyl]-2-hydroxymethyl-phenol;
- 4-{2-[1-(2,6-Dimethyl-benzyl)-cyclopropylamino]-1-hydroxy-ethyl}-2-hydroxymethyl-phenol;
- 6-Hydroxy-8-{1-hydroxy-2-[1-(4-methoxy-benzyl)-cyclopropylamino]-ethyl}-4H-benzo[1,4]oxazin-3-on;
- 6-Hydroxy-8-{1-hydroxy-2-[1-(4-hydroxy-benzyl)-cyclopropylamino]-ethyl}-4H-benzo[1,4]oxazin-3-on;
- 6-Hydroxy-8-{1-hydroxy-2-[(1-phenyl-cyclopropylmethyl)-amino]-ethyl}-4H-benzo[1,4]oxazin-3-on;
- 8-{2-[1-(2,6-Dimethyl-benzyl)-cyclopropylamino]-1-hydroxy-ethyl}-6-hydroxy-4H-benzo[1,4]oxazin-3-on;
- 8- {2-[1-(4-Chlor-phenoxymethyl)-cyclopropylamino]-1-hydroxy-ethyl}-6-hydroxy-4H-benzo[1,4]oxazin-3-on;
- 1-(4-Amino-3,5-dichlor-phenyl)-2-[(1-phenyl-cyclopropylmethyl)-amino}-ethanol;
- 1-(4-Amino-3,5-dichlor-phenyl)-2-[1-(2,6-dimethyl-benzyl)-cyclopropylamino]-ethanol;
- 1-(4-Amino-3,5-dichlor-phenyl)-2-[1-(4-methoxy-benzyl)-cyclopropylamino]-ethanol;
- 1-(4-Amino-3,5-dichlor-phenyl)-2-(1-benzyl-cyclopropylamino)-ethanol;
- 8-Hydroxy-5-{1-hydroxy-2-[1-(4-methoxy-benzyl)-cyclopropylamino]-ethyl}-1H-chinolin-2-on und
- 8-Hydroxy-5-{1-hydroxy-2-[1-(4-trifluormethyl-benzyl)-cyclopropylamino]-ethyl}-1H-chinolin-2-on,
gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

Die Verbindungen der Formel **1** können gegebenenfalls in Form der einzelnen optischen Isomeren, Mischungen der einzelnen Enantiomeren oder Racemate zur Anwendung gelangen. Besonders bevorzugt gelangen Sie in Form der enantiomerenreinen Verbindungen zur Anwendung, wobei die Verbindungen der Formel **1**, in denen das zum Phenylring benzylische, asymmetrische Kohlenstoffzentrum "-CH(OH)-" R-konfiguriert ist. Die besonders bevorzugten *R*-Enantiomere der Verbindungen der allgemeinen Formel **1** sind durch die allgemeine Formel ***R*****-****1** darstellbar, 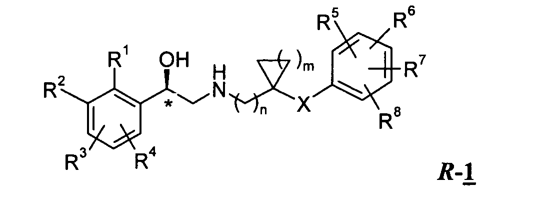 worin die Gruppen R¹, R², R³, R⁴, R⁵, R⁶, R⁷, R⁸, n, m und X die vorstehend genannten Bedeutungen haben können.

Unter Säureadditionssalzen mit pharmakologisch unbedenklichen Säuren werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-*p*-toluolsulfonat, bevorzugt Hydrochlorid, Hydrobromid, Hydrosulfat, Hydrosuccinat, Hydrofumarat und Hydromethansulfonat verstanden.

Halogen steht im Rahmen der vorliegenden Erfindung für Fluor, Chlor, Brom oder Jod. Sofern nicht gegenteilig angegeben, gelten Fluor und Chlor als bevorzugte Halogene, wobei Fluor im Allgemeinen bevorzugt ist.

Als Alkylgruppen (Alkyl) werden, soweit nicht anders angegeben, verzweigte und unverzweigte Alkylgruppen mit 1 bis 6, bevorzugt 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Als Alkylengruppen (Alkylen) werden, soweit nicht anders angegeben, verzweigte und unverzweigte Alkylengruppen mit 1 bis 6, bevorzugt 1 bis 4 Kohlenstoffatomen bezeichnet. Beispielsweise werden genannt: Methylen, Ethylen, Propylen oder Butylen. Sofern nicht anders beschrieben, umfassen die Definitionen Propylen und Butylen alle denkbaren isomeren Formen der jeweiligen Reste.

Als Alkenylengruppen (Alkenylen) werden, soweit nicht anders angegeben, verzweigte und unverzweigte Alkenylengruppen mit 1 bis 6, bevorzugt 1 bis 4 Kohlenstoffatomen bezeichnet. Beispielsweise werden genannt: Ethenylen, Propenylen oder Butenylen.

Als Cycloalkylgruppen (Cycloalkyl) werden, soweit nicht anders angegeben cyclische Alkylgruppen mit 3 bis 6 bezeichnet. Beispielsweise werden genannt: Cyclopropyl, Cyclobutanyl, Cyclopentyl oder Cyclohexyl. Hydroxyalkylgruppen sind im Rahmen der vorliegenden Erfindung, Cycloalkylgruppen, in denen ein oder mehrere, bevorzugt ein Wasserstoffatom durch Hydroxy substituiert ist.

Als Alkyloxygruppen (O-Alkyl) werden, soweit nicht anders angegeben, verzweigte und unverzweigte Alkylgruppen mit 1 bis 6, bevorzugt 1 bis 4 Kohlenstoffatomen bezeichnet, die über ein Sauerstoffatom verknüpft sind. Beispielsweise werden genannt: Methyloxy, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen -OMe, -OEt, -OProp oder -OBu verwendet. Sofern nicht anders beschrieben, umfassen die Definitionen Propyloxy und Butyloxy alle denkbaren isomeren Formen der jeweiligen Reste. So umfasst beispielsweise Propyloxy *n*-Propyloxy und *iso*-Propyloxy, Butyloxy umfasst *iso*-Butyloxy, *sec*-Butyloxy und *tert*-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Als Halogenalkylengruppen (Haloalkyl) werden, soweit nicht anders angegeben, verzweigte und unverzweigte Alkylgruppen mit 1 bis 6 Kohlenstoffatomen bezeichnet, bei denen ein oder mehrere Wasserstoffatome durch Halogenatome, bevorzugt durch Fluor ersetzt sind. Beispielsweise werden genannt: CHF₂, CF₃, CH₂CF₃, CF₂CF₃.

Als Arylgruppen werden, soweit nicht anders angegeben, aromatische Ringsysteme mit 6 bis 10 Kohlenstoffatomen bezeichnet. Bevorzugte Arylgruppen sind Phenyl und Naphthyl, wobei Phenyl erfindungsgemäß besonders bevorzugt ist.

Als heterocyclische Gruppen (Heterocyclus) werden, soweit nicht anders angegeben, aromatische oder nicht aromatische Ringsysteme mit 2 bis 5 Kohlenstoffatomen und 1, 2 oder 3 Atomen ausgewählt aus der Gruppe O, S oder N, bevorzugt N, bezeichnet. Besonders bevorzugte Heterocyclen sind Piperidin, Piperazin, Morpholin, Pyrolidin, Pyrrol, Imidazol, Triazol, Pyridin, Pyrimidin, Thiophen, Tetrahydrofuran oder Furan.

Die Herstellung der erfindungsgemäßen Verbindungen kann in Analogie zu im Stand der Technik bereits bekannten Vorgehensweisen erfolgen. Geeignete Herstellverfahren sind beispielsweise aus der WO95/33724 bekannt, auf die an dieser Stelle vollinhaltlich Bezug genommen wird.

Wesentliche Bausteine zur Herstellung der erfindungsgemäßen Verbindungen der allgemeinen Formel **1** sind insbesondere die Cycloalkylamine der Formel **2**, 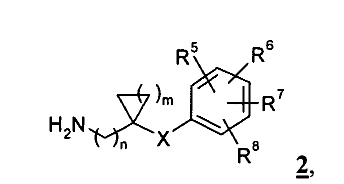 worin die Gruppen R⁵, R⁶, R⁷, R⁸, n, m und X die vorstehend genannten Bedeutungen haben können.

Die Verbindungen der Formel **2** sind in Analogie zu im Stand der Technik bekannten Methoden herstellbar (Bertus et al., J. Org. Chem. 2002, 67, 3965-3968).

Die Verbindungen der Formel **2** sind noch nicht im Stand der Technik bekannt. Dementsprechend betrifft ein weiterer Aspekt der vorliegenden Erfindung, die Verbindungen der Formel **2**,
worin
- n: 0 oder 1;
- m: 1;
- X: eine Einfachbindung oder eine der zweibindigen Gruppen -O-, -NH, -S-, C₂-C₆- Alkenylen, -O-C₁-C₆-Alkylen, -NH-C₁-C₆-Alkylen, -S-C₁-C₆-Alkylen oder C₁-C₆- Alkylen;
- R⁵, R⁶, R⁷ und R⁸,: gleich oder verschieden, Wasserstoff, OR⁹, -C₁-C₆-Alkyl, -C₁-C₆-Haloalkyl, -C₁-C₆-Hydroxyalkyl, -C₃-C₆-Cycloalkyl, -C₃-C₆-Hydroxycycloalkyl, -CN, NO₂, -COR⁹, -COOR⁹, -CONR¹⁰R¹¹, -NR¹⁰R¹¹, -NR¹⁰COR⁹, -NR¹⁰SO₂R¹², -SR¹², -SOR¹², -SO₂R¹², -SO₂NR¹⁰R¹¹ oder Halogen, oder
zwei der Reste R⁵, R⁶, R⁷ und R⁸ bilden, sofern sie am substituierenden Phenylring vicinal ständig sind, gemeinsam eine zweibindige Gruppe ausgewählt aus C₂-C₆-Alkylen, C₂-C₆-Alkenylen und -O-C₁-C₆-alkylen-O- in der jeweils ein oder 2 Wasserstoffatome ersetzt sein können durch einen oder zwei Reste ausgewählt aus der Gruppe bestehend aus -C₁-C₄-Alkyl, -C₁-C₄-Alkoxy, OH, oder Halogen;
- R⁹: Wasserstoff, C₁-C₄-Alkyl, C₆-C₁₀-Aryl oder C₆-C₁₀-Aryl-C₁-C₄-alkylen;
- R¹⁰ und R¹¹: gleich oder verschieden, Wasserstoff, C₁-C₄-Alkyl, C₆-C₁₀-Aryl oder C₆-C₁₀-Aryl-C₁-C₄-alkylen;
- R¹²: C₁-C₄-Alkyl, C₆-C₁₀-Aryl oder C₆-C₁₀-Aryl-C₁-C₄-alkylen;
bedeuten, gegebenenfalls in Form der einzelnen optischen Isomeren, Mischungen der einzelnen Enantiomeren oder Racemate.

Bevorzugt sind Verbindungen der allgemeinen Formel **2**,
worin
- n: 0 oder 1, bevorzugt 0;
- m: 1;
- X: eine Einfachbindung oder eine der zweibindigen Gruppen C₂-C₄-Alkenylen, -O-C₁-C₄-Alkylen, -NH-C₁-C₄-Alkylen, -S-C₁-C₄-Alkylen oder C₁-C₄-Alkylen;
- R⁵, R⁶, R⁷ und R⁸,: gleich oder verschieden, Wasserstoff, OR⁹, -C₁-C₄-Alkyl, -C₁-C₄-Haloalkyl, -C₁-C₄-Hydroxyalkyl, -C₃-C₆-Cycloalkyl, -C₃-C₆-Hydroxycycloalkyl, -CN, NO₂, -COR⁹, -COOR⁹, -CONR¹⁰R¹¹, -NR¹⁰R¹¹, -NR¹⁰COR⁹, -NR¹⁰SO₂R¹², -SR¹², -SOR¹², -SO₂R¹², -SO₂NR¹⁰R¹¹, Fluor, Chlor oder Brom, oder
zwei der Reste R⁵, R⁶, R⁷ und R⁸ bilden, sofern sie am substituierenden Phenylring vicinal ständig sind, gemeinsam eine zweibindige Gruppe ausgewählt aus C₂-C₄-Alkylen, C₂-C₄-Alkenylen und -O-C₁-C₄-Alkylen-O- in der jeweils ein oder 2 Wasserstoffatome ersetzt sein können durch einen oder zwei Reste ausgewählt aus der Gruppe bestehend aus Methyl, Methoxy, OH, Fluor, Chlor oder Brom;
- R⁹: Wasserstoff, Methyl, Ethyl, Phenyl, Naphthyl, Benzyl, Naphthylmethyl oder 2- Phenylethyl;
- R¹⁰ und R¹¹: gleich oder verschieden, Wasserstoff, Methyl, Ethyl, Phenyl, Naphthyl, Naphthylmethyl, Benzyl oder 2-Phenylethyl;
- R¹²: Methyl, Ethyl, Phenyl, Naphthyl, Naphthylmethyl, Benzyl oder 2-Phenylethyl;
bedeuten, gegebenenfalls in Form der einzelnen optischen Isomeren, Mischungen der einzelnen Enantiomeren oder Racemate.

Besonders bevorzugt sind Verbindungen der allgemeinen Formel **2**,
worin
- n: 0 oder 1, bevorzugt 1;
- m: 1;
- X: eine Einfachbindung oder eine der zweibindigen Gruppen -CH₂-, -CH₂-CH₂-, -CH₂-CH₂-CH₂-, -CH=CH-, -CH₂-CH=CH-, -CH₂-O-, -CH₂-CH₂-O-, -CH₂-O-, -CH₂-CH₂-O-, -CH₂-NH₂-, -CH₂-CH₂-NH₂-;
- R⁵, R⁶, R⁷ und R⁸,: gleich oder verschieden, Wasserstoff, OH, Methyl, Ethyl, Propyl, Butyl, -CH₂F, -CHF₂, -CF₃, -CH₂-CH₂F, -CH₂-CHF₂, -CH₂-CF₃, -CH₂-CH₂-OH, -CH₂-OH, Cyclpropyl, Cyclobutyl, Cyclopentyl, HO-Cyclpropyl, HO-Cyclobutyl, HO-Cyclopentyl, -CN, NO₂, -COPhenyl, -COOMethyl, -COOEthyl, -CONH₂, -CONHMethyl, -CONHPhenyl, -CONHBenzyl, -CON(Methyl)₂, NH₂, NH(Methyl), N(Methyl)₂, -NHCOMethyl, -NHCOPhenyl, -NHSO₂Methyl, -NHSO₂Phenyl, -NHSO₂-Phenyl-CH₃, -SO₂Methyl, -SO₂-Phenyl, -SO₂-Phenyl-CH₃, -SO₂NH₂, Fluor, Chlor oder Brom, oder
zwei der Reste R⁵, R⁶, R⁷ und R⁸ bilden, sofern sie am substituierenden Phenylring vicinal ständig sind, gemeinsam eine zweibindige Gruppe ausgewählt aus -CH₂- CH₂-CH₂-, -CH₂-CH₂-CH₂-CH₂-, -CH₂-CH=CH-, -CH=CH-CH=CH-, -O-CH₂-O- und -O-CH₂-CH₂-O- in der jeweils ein oder 2 Wasserstoffatome ersetzt sein können durch einen oder zwei Reste ausgewählt aus der Gruppe bestehend aus Methyl, Methoxy, OH oder Fluor,
bedeuten, gegebenenfalls in Form der einzelnen optischen Isomeren, Mischungen der einzelnen Enantiomeren oder Racemate.

Besonders bevorzugt sind Verbindungen der Formel **2**, worin
- X: eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-, bedeutet
und worin die Gruppen R⁵, R⁶, R⁷, R⁸, n und m vorstehend genannte Bedeutungen haben können, gegebenenfalls in Form der einzelnen optischen Isomeren, Mischungen der einzelnen Enantiomeren oder Racemate.

Besonders bevorzugt sind ferner Verbindungen der Formel **2**, worin
- R⁵, R⁶, R⁷ und R⁸,: gleich oder verschieden, Wasserstoff, OH, Methyl, Ethyl, Propyl, Butyl, CHF₂, CH₂F, CF₃, -CH₂-CH₂-OH, -CH₂-OH, Methyloxy, Ethyloxy, Propyloxy, Butyloxy, Cyclopropyl, Hydroxycyclopropyl, NH₂, NHMethyl, N(Methyl)₂ Fluor, Chlor oder Brom, oder
zwei der Reste R⁵, R⁵, R⁷ und R⁸ gemeinsam, sofern sie am substituierenden Phenylring vicinal ständig sind, die zweibindige Gruppe -CH=CH-CH=CH- in der jeweils ein oder 2 Wasserstoffatome ersetzt sein können durch einen oder zwei Reste ausgewählt aus der Gruppe bestehend aus Methyl, Methoxy, OH, Fluor, Chlor oder Brom, bedeuten,
und worin die Gruppen n, m und X vorstehend genannte Bedeutungen haben können, gegebenenfalls in Form der einzelnen optischen Isomeren, Mischungen der einzelnen Enantiomeren oder Racemate.

Besonders bevorzugt sind ferner Verbindungen der Formel **2**, worin
- R⁵, R⁶, R⁷ und R⁸,: gleich oder verschieden, Wasserstoff, OH, Methyl, Ethyl, Propyl, Butyl, CHF₂, CH₂F, CF₃, Methyloxy, Ethyloxy, Propyloxy, Butyloxy, Cyclopropyl, Hydroxycyclopropyl, NH₂, Fluor, Chlor oder Brom,
bedeuten und worin die Gruppen n, m und X vorstehend genannte Bedeutungen haben können, gegebenenfalls in Form der einzelnen optischen Isomeren, Mischungen der einzelnen Enantiomeren oder Racemate.

Besonders bevorzugt sind ferner Verbindungen der Formel **2**, worin
- R⁵, R⁶, R⁷ und R⁸,: gleich oder verschieden, Wasserstoff, OH, Methyl, CF₃, Methyloxy, Ethyloxy, Propyloxy, Butyloxy, Hydroxycyclopropyl, NH₂, Fluor oder Chlor,
bedeuten und worin die Gruppen n, m und X vorstehend genannten Bedeutungen haben können, gegebenenfalls in Form der einzelnen optischen Isomeren, Mischungen der einzelnen Enantiomeren oder Racemate.

Besonders bevorzugt sind ferner Verbindungen der Formel **2**, worin
- R⁵ und R⁸: Wasserstoff;
- R⁶ und R⁷: gemeinsam, sofern sie am substituierenden Phenylring vicinal ständig sind, die zweibindige Gruppe -CH=CH-CH=CH- bedeuten,
und worin die Gruppen n, m und X vorstehend genannte Bedeutungen haben können, gegebenenfalls in Form der einzelnen optischen Isomeren, Mischungen der einzelnen Enantiomeren oder Racemate.

Ein bevorzugter Aspekt der vorliegenden Erfindung betrifft ferner Verbindungen der allgemeinen Formel **2**, worin
- n: 0 oder 1, bevorzugt 0;
- m: 1;
- X: eine Einfachbindung oder eine der zweibindigen Gruppen -CH₂-, -CH₂-CH₂-, -CH₂-CH₂-CH₂-, -CH=CH-, -CH₂-CH=CH-, -CH₂-O-, -CH₂-CH₂-O-, -CH₂-NH₂-, -CH₂-CH₂-NH₂-;
- R⁵ und R⁸: gleich oder verschieden, Wasserstoff, Methyl, Methyloxy oder Fluor;
- R⁶ und R⁷: gleich oder verschieden, Wasserstoff, OH, Methyl, Ethyl, CF₃, Methyloxy, Ethyloxy, Propyloxy, Butyloxy, Cyclopropyl, Hydroxycyclopropyl, NH₂ oder Fluor,
oder
R⁶ und R⁷, sofern sie am substituierenden Phenylring vicinal ständig sind, gemeinsam die zweibindige Gruppe -CH=CH-CH=CH- in der jeweils ein oder 2 Wasserstoffatome ersetzt sein können durch einen oder zwei Reste ausgewählt aus der Gruppe bestehend aus Methyl, Methoxy, OH, Fluor, Chlor und Brom,
bedeuten, gegebenenfalls in Form der einzelnen optischen Isomeren, Mischungen der einzelnen Enantiomeren oder Racemate.

Bevorzugt sind Verbindungen der allgemeinen Formel **2**, worin
- n: 0 oder 1, bevorzugt 0;
- m: 1;
- X: eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-,
- R⁵ und R⁸: gleich oder verschieden, Wasserstoff, Methyl, Methyloxy oder Fluor;
- R⁶ und R⁷: gleich oder verschieden, Wasserstoff, OH, Methyl, CF₃, Methyloxy, Ethyloxy, Propyloxy, Butyloxy, Hydroxycyclopropyl, NH₂ oder Fluor,
oder
R⁶ und R⁷, sofern sie am substituierenden Phenylring vicinal ständig sind, gemeinsam die zweibindige Gruppe -CH=CH-CH=CH-,
bedeuten, gegebenenfalls in Form der einzelnen optischen Isomeren, Mischungen der einzelnen Enantiomeren oder Racemate.

Besonders bevorzugt sind Verbindungen der allgemeinen Formel **2**, worin
- R⁵, R⁶ und R⁸: gleich oder verschieden, Wasserstoff oder Methyl, bevorzugt Wasserstoff;
- R⁷: bevorzugt Wasserstoff, OH, Methyl, CF₃, Methyloxy, Ethyloxy, Propyloxy oder Butyloxy, OH, Methyloxy, Ethyloxy, Propyloxy oder Butyloxy,
bedeuten, und n, m und X die vorstehend genannten Bedeutungen haben können, gegebenenfalls in Form der einzelnen optischen Isomeren, Mischungen der einzelnen Enantiomeren oder Racemate.

Besonders bevorzugte Verbindungen der Formel **2** sind ausgewählt aus der Gruppe bestehend aus:

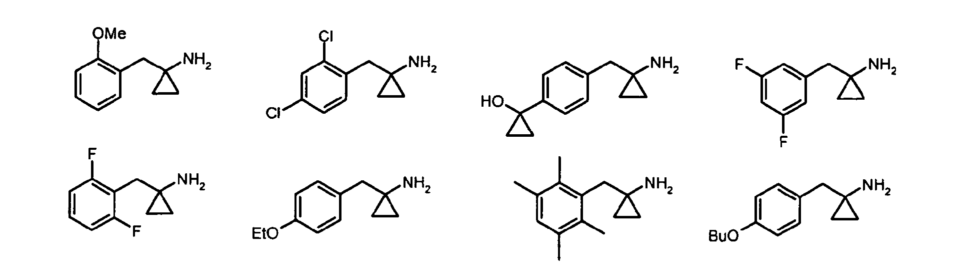

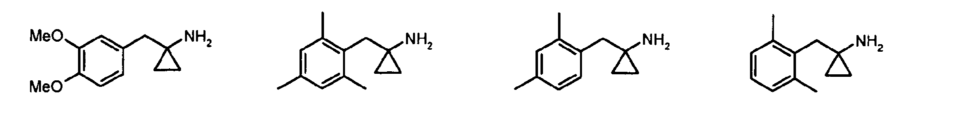

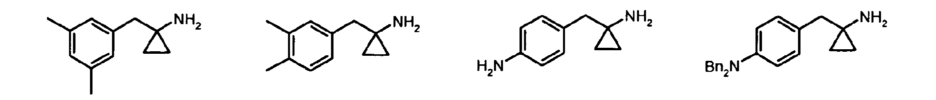

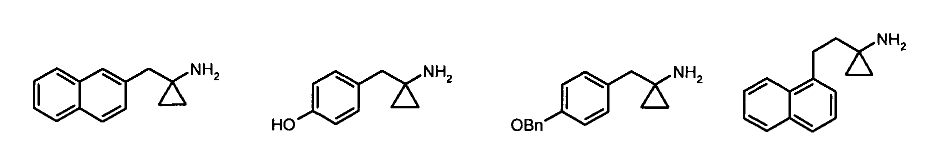

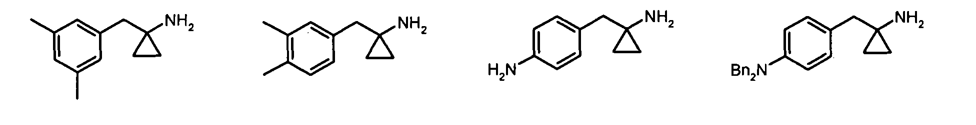

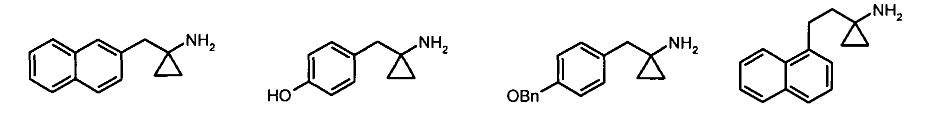

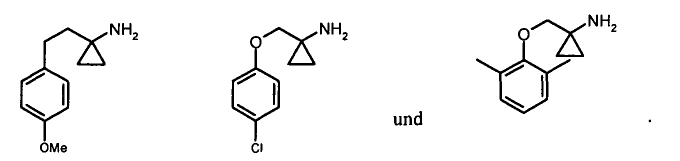

Die nachstehend beschriebenen Synthesebeispiele dienen der weitergehenden Illustration von neuen erfindungsgemäßen Verbindungen. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

### Synthese der Zwischenstufen:

### Allgemeine Arbeitsvorschrift 1:

Zu einer Lösung von 1 Äquivalent (typischerweise ungefähr 30 mmol) des Phenylactonitrils in 150 mL Diethylether wird bei -15 bis -25°C 1 Äquivalent Titantetraisopropylat getropft. Es wird 30 Minuten bei Raumtemperatur gerührt und dann mit 2 Äquivalenten Bortrifluorid-Diethyletherat versetzt, wobei die Reaktionsmischung so gekühlt wird, dass die Temperatur nicht über 20°C steigt. Man läßt 30 Minuten rühren, tropft langsam 120 mL 1 M Natronlauge zu und rührt weitere 60 Minuten. Die wässrige Phase wird abgetrennt und mit Diethylether ausgeschüttelt. Anschließend werden die vereinigten Etherphasen mit Natriumsulfit-Lösung gewaschen und mit 0.5 molarer Salzsäure extrahiert. Die das Produkt enthaltende Salzsäurefraktion wird mit Natronlauge alkalisch gestellt und mit Dichlormethan extrahiert. Die organischen Phasen werden mit Natriumsulfat getrocknet und eingeengt. Der Rückstand wird mittels Chromatographie an einer Kieselgelsäule (Dichlormethan/Methanol/Ammoniak-Gradient) gereinigt.

### 1-(2,3,5,6-Tetramethyl-benzyl)-cyclopropylamin

Herstellung aus 5.0 g (29 mmol) (2,3,5,6-Tetramethyl-phenyl)-acetonitril nach der allgemeinen Arbeitsvorschrift 1.

Ausbeute: 2.5 g (43%); Massenspektroskopie: [M+H]⁺ = 204.

### 1-(3,4-Dimethoxy-benzyl)-cyclopropylamin

Die Verbindung wird nach der allgemeinen Arbeitsvorschrift 1 aus 5.2 g (29 mmol) (3,4-Dimethoxy-phenyl)-acetonitril hergestellt. Ausbeute: 1.6 g (27%); Massenspektroskopie: [M+H]⁺ = 208.

### 1-(4-Butoxy-benzyl)-cyclopropylamin

Herstellung nach der allgemeinen Arbeitsvorschrift 1 aus 5.6 g (29 mmol) (4-Butoxyphenyl)-acetonitril. Ausbeute: 2.7 g (43%); Massenspektroskopie: [M+H]⁺ = 220.

### 1-(4-Ethoxy-benzyl)-cyclopropylamin

Herstellung nach der allgemeinen Arbeitsvorschrift 1 aus 4.7 g (29 mmol) (4-Ethoxyphenyl)-acetonitril. Ausbeute: 1.9 g (35%); Massenspektroskopie: [M+H]⁺= 192.

### 1-(2,6-Difluor-benzyl)-cyclopropylamin

Herstellung in Analogie zur allgemeinen Arbeitsvorschrift 1 aus 5.0 g (33 mmol) (2,6-Difluor-phenyl)-acetonitril. Ausbeute: 3.0 g (50%); Massenspektroskopie: [M+H]⁺ = 184.

### 1-(4-Chlor-benzyl)-cyclopropylamin

Herstellung nach der allgemeinen Arbeitsvorschrift 1 aus 4.5 g (29 mmol) (4-Chlorphenyl)-acetonitril. Ausbeute: 2.0 g (38%); Massenspektroskopie: [M+H]⁺ = 182/4.

### 1-[4-(1-Amino-cyclopropylmethyl)-phenyl]-cyclopropanol

4.0 g (21 mmol) 4-Cyanomethyl-benzoesäureethylester werden in Analogie zur allgemeinen Arbeitsvorschrift 1 umgesetzt und aufgearbeitet. Ausbeute: 0.6 g (14%); Massenspektroskopie: [M+H]⁺ = 204.

### 1-(3,5-Difluor-benzyl)-cyclopropylamin

Herstellung nach der allgemeinen Arbeitsvorschrift 1 aus 5.0 g (32 mmol) (3,5-Difluorphenyl)-acetonitril. Ausbeute: 2.7 g (47%); Massenspektroskopie: [M+H]⁺ = 184.

### 1-(4-Trifluormethyl-benzyl)-cyclopropylamin

Die Verbindung wird nach der allgemeinen Arbeitsvorschrift 1 aus 5.0 g (26 mmol) (4-Trifluormethyl-phenyl)-acetonitril hergestellt. Ausbeute: 3.1 g (54%); Massenspektroskopie: [M+H]⁺ = 216.

### 1-(2,4-Dichlor-benzyl)-cyclopropylamin

Herstellung nach der allgemeinen Arbeitsvorschrift 1 aus 6.3 g (33 mmol) (2,4-Dichlorphenyl)-acetonitril. Ausbeute: 3.1 g (43%); Massenspektroskopie: [M+H]⁺ = 216/218/220.

### 1-(2-Chlor-benzyl)-cyclopropylamin

Herstellung nach der allgemeinen Arbeitsvorschrift 1 aus 5.1 g (34 mmol) (2-Chlorphenyl)-acetonitril. Ausbeute: 2.3 g (38%); Massenspektroskopie: [M+H]⁺ = 182/184.

### 1-(4-Trifluormethoxy-benzyl)-cyclopropylamin

Herstellung nach der allgemeinen Arbeitsvorschrift 1 aus 5.0 g (25 mmol) (4-Trifluormethoxy-phenyl)-acetonitril. Ausbeute: 2.9 g (51 %); Massenspektroskopie: [M+H]⁺ = 232.

### 1-(2-Methoxy-benzyl)-cyclopropylamin

Herstellung nach der allgemeinen Arbeitsvorschrift 1 aus 5.0 g (34 mmol) (2-Methoxyphenyl)-acetonitril. Ausbeute: 1.7 g (28%); Massenspektroskopie: [M+H]⁺ = 178.

### 1-(2,6-Dimethyl-benzyl)-cyclopropylamin

Die Verbindung wird nach der allgemeinen Arbeitsvorschrift 1 aus 5.8 g (40 mmol) (2,6-Dimethyl-phenyl)-acetonitril hergestellt. Ausbeute: 1.4 g (20%); Massenspektroskopie: [M+H]⁺ = 176.

### 1-(2,4,6-Trimethyl-benzyl)-cyclopropylamin

Herstellung nach der allgemeinen Arbeitsvorschrift 1 aus 5.0 g (31 mmol) (2,4,6-Trimethyl-phenyl)-acetonitril. Ausbeute: 2.2 g (37%); Massenspektroskopie: [M+H]⁺ = 190.

### 1-(2,4-Dimethyl-benzyl)-cyclopropylamin

Herstellung nach der allgemeinen Arbeitsvorschrift 1 aus 5.0 g (34 mmol) (2,4-Dimethylphenyl)-acetonitril. Ausbeute: 1.9 g (32%); Massenspektroskopie: [M+H]⁺ = 176.

### 1-(2,6-Dichlor-benzyl)-cyclopropylamin

Herstellung nach der allgemeinen Arbeitsvorschrift 1 aus 5.2 g (27 mmol) (2,6-Dichlorphenyl)-acetonitril. Ausbeute: 2.7 g (46%); Massenspektroskopie: [M+H]⁺ = 216/218/220.

### 1-(2-Trifluormethyl-benzyl)-cyclopropylamin

Das Amin wird nach der allgemeinen Arbeitsvorschrift 1 aus 5.0 g (27 mmol) (2-Trifluormethyl-phenyl)-acetonitril hergestellt. Ausbeute: 2.8 g (48%); Massenspektroskopie: [M+H]⁺ = 216.

### 1-(3-Methyl-benzyl)-cyclopropylamin

Herstellung nach der allgemeinen Arbeitsvorschrift 1 aus 5.4 g (40 mmol) m-Tolylacetonitril. Ausbeute: 1.7 g (26%); Massenspektroskopie: [M+H]⁺ = 162.

### 1-(2-Methyl-benzyl)-cyclopropylamin

Herstellung nach der allgemeinen Arbeitsvorschrift 1 aus 3.5 mL (28 mmol) o-Tolylacetonitril. Ausbeute: 0.58 g (13%); Massenspektroskopie: [M+H]⁺ = 162.

### 1-(3,5-Dimethyl-benzyl)-cyclopropylamin

Herstellung nach der allgemeinen Arbeitsvorschrift 1 aus 5.9 g (40 mmol) (3,5-Dimethylphenyl)-acetonitril. Ausbeute: 3.8 g (54%); Massenspektroskopie: [M+H]⁺ = 176.

### 1-(3,4-Dimethyl-benzyl)-cyclopropylamin

Das Amin wird nach der allgemeinen Arbeitsvorschrift 1 aus 5.9 g (40 mmol) (3,4-Dimethyl-methyl-phenyl)-acetonitril erhalten. Ausbeute: 2.4 g (34%); Massenspektroskopie: [M+H]⁺ = 176.

### 1-Benzyl-cyclopropylamin

Herstellung nach der allgemeinen Arbeitsvorschrift 1 aus 4.7 g (40 mmol) Phenylacetonitril. Ausbeute: 2.3 g (39%); Massenspektroskopie: [M+H]⁺ = 148.

### 1-(4-Methoxy-benzyl)-cyclopropylamin

Das Amin wird nach der allgemeinen Arbeitsvorschrift 1 aus 5.5 mL (40 mmol) (4-Methoxy-phenyl)-acetonitril erhalten. Ausbeute: 4.2 g (59%); Massenspektroskopie: [M+H]⁺ = 178.

### 1-Phenoxymethyl-cyclopropylamin

Das Amin wird nach der allgemeinen Arbeitsvorschrift 1 aus 3.94 g (29 mmol) Phenoxyacetonitril erhalten. Ausbeute: 1.80 g (38%); Massenspektroskopie: [M+H]⁺ = 164.

### 1-(2,6-Dimethyl-phenoxymethyl)-cyclopropylamin

Das Amin wird nach der allgemeinen Arbeitsvorschrift 1 aus 4.80 g (30 mmol) (2,6-Dimethyl-phenoxy)-acetonitril erhalten. Ausbeute: 2.56 g (45%); Massenspektroskopie: [M+H]⁺ = 192.

### 1-[2-(4-Methoxy-phenyl)-ethyl]-cyclopropylamin

Herstellung nach der allgemeinen Arbeitsvorschrift 1 aus 5.00 g (31 mmol) 3-(4-Methoxyphenyl)-propionnitril. Ausbeute: 1.90 g (32%); Massenspektroskopie: [M+H]⁺ = 192.

### 1-Naphthalen-2-ylmethyl-cyclopropylamin

Das Amin wird nach der allgemeinen Arbeitsvorschrift 1 aus 5.75 g (33 mmol) Naphthalen-2-yl-acetonitril hergestellt. Ausbeute: 2.30 g (35%); Massenspektroskopie: [M+H]⁺ = 198.

### 1-(4-Benzyloxy-benzyl)-cyclopropylamin

Die Herstellung erfolgt in Analogie zur allgemeinen Arbeitsvorschrift 1 aus 25 g (112 mmol) Naphthalen-2-yl-acetonitril. Ausbeute: 14.1 g (50%); Massenspektroskopie: [M+H]⁺ = 254.

### 4-( 1-Amino-cyclopropylmethyl)-phenol

Hydrierung von 5.00 g (19.7 mmol) 1-(4-Benzyloxy-benzyl)-cyclopropylamin in 60 mL Methanol bei 3 bar mit Palladium auf Kohle als Katalysator. Ausbeute: 2.25 g (70%); Massenspektroskopie: [M+H]⁺ = 164.

### 1-(2-Naphthalen-1-yl-ethyl)-cyclopropylamin

Das Amin wird nach der allgemeinen Arbeitsvorschrift 1 aus 5.4 g (30 mmol) 3-Naphthalen-1-yl-propionitril hergestellt. Ausbeute: 2.2 g (35%); Massenspektroskopie: [M+H]⁺ = 212.

### 1-(4-Chlor-phenoxymethyl)-cyclopropylamin

Das Amin wird nach der allgemeinen Arbeitsvorschrift 1 aus 4.86 g (29 mmol) (4-Chlorphenoxy)-acetonitril hergestellt. Ausbeute: 2.7 g (47%); Massenspektroskopie: [M+H]⁺ = 198/200.

### [4-(1-Amino-cyclopropylmethyl)-phenyl]-dibenzyl-amin

Umsetzung von 32 g (102 mmol) (4-Dibenzylamino-phenyl)-acetonitril nach der allgemeinen Arbeitsvorschrift 1. Ausbeute: 14.1 g (40%); Massenspektroskopie: [M+H]⁺ = 343.

### 4-(1-Amino-cyclopropylmethyl)-phenylamin

3.0 g (8.8 mmol) [4-(1-Amino-cyclopropylmethyl)-phenyl]-dibenzyl-amin werden in 30 mL Methanol gelöst und bei 3 bar in Gegenwart von Palladium auf Kohle hydriert. Anschließend wird der Katalysator abfiltriert und das Filtrat eingeengt. Der Rückstand wird in Diethylether gelöst und mit 4 molarer Salzsäure in Dioxan versetzt. Nach dem Abdestillieren des Lösungsmittels wird das Hydrochlorid zur weiteren Reinigung in Diisopropylether suspendiert. Ausbeute: 1.5 g (86%; Hydrochlorid); Massenspektroskopie: [M+H]⁺ = 163/65.

### Allgemeine Arbeitsvorschrift 2 (Synthese von Cyclopentyl- und Cyclohexylaminen)

Eine Lösung von 1 Äquivalent (üblicherweise 70 mmol) Keton in 70 mL THF wird bei - 20°C zu 1.1 Äquivalenten Grignard-Reagenz in THF getropft. Nach 30 Minuten Rühren bei Raumtemperatur wird die Reaktion durch Zugabe von Ammoniumchlorid-Lösung beendet und man extrahiert mit Dichlormethan. Die organischen Phasen werden mit Natriumsulfat getrocknet und vom Lösungsmittel beftreit. Der Rückstand wird ohne weitere Reinigung direkt weiter umgesetzt.

Hierzu werden der Alkohol und 8.7 mL (69.5 mmol) Trimethylsilylcyanid bei 10°C mit 10.7 mL Essigsäure versetzt. Anschließend tropft man 14 mL konz. Schwefelsäure so zu, dass die Temperatur nicht über 20°C steigt. Nach zwei Stunden Rühren bei Raumtemperatur werden 140 mL 6 molare Natronlauge und 100 mL tert-Butylether zugefügt. Die organische Phase wird abgetrennt, mit Natriumsulfat getrocknet und eingeengt. Das als Öl vorliegende Formamid wird in 16 mL Ethanol gelöst, mit 50 mL einer 20 molaren Natronlauge versetzt und über Nacht refluxiert. Nach Abkühlung auf Raumtemperatur und Zugabe von Toluol werden die Phasen getrennt. Die organische Phase wird mit Wasser gewaschen, mit Natriumsulfat getrocknet und vom Lösungsmittel befreit. Der Rückstand wird in Ethylacetat gelöst und mit 10%iger Salzsäure in Ethylacetat versetzt bis zur sauren Reaktion. Das ausfallende Hydrochlorid wurde abfiltriert und getrocknet.

### 1-Phenethyl-cyclohexylamin

Hergestellt aus 2.0 g (20.4 mmol) Cyclohexanon und Phenethylmagnesiumchlorid (1 molare Lösung in Tetrahydrofuran) nach der allgemeinen Arbeitsvorschrift 5. Ausbeute: 1.44 g (29%; Hydrochlorid). Massenspektroskopie: [M+H]⁺ = 204.

### 1-Benzyl-cyclopentylamin

Hergestellt aus 2.3 g (27.3 mmol) Cyclopentanon und Benzylmagnesiumchlorid (2 molare Lösung in Tetrahydrofuran) nach der allgemeinen Arbeitsvorschrift 5. Ausbeute: 1.56 g (27%; Hydrochlorid). Massenspektroskopie: [M+H]⁺ = 176.

### 1-(4-Fluorobenzyl)-cyclopentylamin

Hergestellt aus 2.7 g (32.1 mmol) Cyclopentanon und 4-Fluorbenzylmagnesiumchlorid (0.25 molare Lösung in Tetrahydrofuran) nach der allgemeinen Arbeitsvorschrift 5. Ausbeute: 3.72 g (50%; Hydrochlorid). Massenspektroskopie: [M+H]⁺ = 194.

### 1-[2-(4-Methoxyphenyl)-ethyl]-cyclohexylamin

Hergestellt aus 4.0 g (40.7 mmol) Cyclohexanon und 4-Methoxyphenethylmagnesiumchlorid (2 molare Lösung in Tetrahydrofuran) nach der allgemeinen Arbeitsvorschrift 5. Ausbeute: 2.53 g (23%; Hydrochlorid). Massenspektroskopie: [M+H]⁺ = 234.

### 1-(4-Methoxy-benzyl)-cyclopentylamin

### 4-(1-Amino-cyclopentylmethyl)-phenylamin

Eine Lösung von 2.1 g (8.4 mmol) 1-Nitro-4-(1-nitrocyclopentylmethyl)-benzol (hergestellt nach der Vorschrift von Hass et. al., *J. Org. Chem.*1949, 71, 2290-2291) in 60 mL Methanol wird mit Raney Nickel als Katalysator bei 3 bar hydriert. Anschließend wird der Katalysator abfiltriert und das Filtrat vom Lösungsmittel befreit. Der Rückstand wird in 15 mL Ethanol gelöst und mit 10%iger Salzäure in Ethanol bis zur sauren Reaktion versetzt. Nach dem Abdestillieren der Lösungsmittel wird der Rückstand in 20 mL Ethanol suspendiert und filtriert. Ausbeute: 1.4 g (Hydrochlorid).

### 1-(4-Methoxy-benzyl)-cyclopentylamin

1.4 g (6.17 mmol) 4-(1-Aminocyclopentylmethyl)-phenylamin Hydrochlorid werden in 25 mL Methanol vorgelegt und bei 0°C mit 2.24 mL (27 mmol) konz. Salzsäure versetzt. Bei dieser Temperatur werden 0.51 g (7.41 mmol) Natriumnitrit in 1 mL Wasser innerhalb von 30 Minuten zugetropft. Nach 2 Stunden Rühren lässt man die Reaktionsmischung auf Raumtemperatur kommen und refluxiert dann über 3 Stunden. Die Lösungsmittel werden abdestilliert und der Rückstand mit Ethylacetat versetzt. Es wird mit wässriger AmmoniakLösung gewaschen, mit Natriumsulfat getrocknet und eingeengt. Der Rückstand wird in Ethanol gelöst und mit 10%iger Salzsäure in Ethanol versetzt. Das als Salz ausfallende Produkt wird abfiltriert, mit Ethanol gewaschen und getrocknet. Ausbeute: 056 g (27% über 2 Stufen, Hydrochlorid), Massenspektroskopie: [M+H]⁺ = 206.

### 1-(4-Methoxy-benzyl)-cyclohexylamin

Erhalten aus 4.7 g (17.8 mmol) of 1-Nitro-4-(1-nitro-cyclohexylmethyl)-benzol (hergestellt nach der Vorschrift von H. B. Hass et al., J. Org. Chem. 1949, 71, 2290-2291) in Analogie zu dem vorstehend beschriebenen Verfahren. Ausbeute: 3.85 g (84%, Hydrochlorid). Massenspektroskopie: [M+H]⁺ = 220.

### 1-[2-(4-Fluor-phenyl)-ethyl]-cyclohexylamin

2.5 g (11.3 mmol) 1-Fluor-4-iod-benzol, 1.5 g (11.9 mmol) 1-Ethinyl-cyclohexylamin, 793 mg (1.13 mmol) PdCl₂(PPh₃)₂, 67 mg (0.35 mmol) Kupferiodid und 50 mL Diisopropylamin werden 2 Stunden bei 70°C gerührt. Anschließend wird die Base abdestilliert und Rückstand mit Ethylacetat versetzt. Die unlöslichen Bestandteile werden abfiltriert und das Filtrat wird eingeengt. Das so hergestellte Alkin wird in 35 mL THF und 35 mL Toluol gelöst und mit Platinoxid als Katalysator bei Normaldruck hydriert. Der Katalysator wird abgetrennt und das Filtrat vom Lösungsmittel befreit. Das Rohprodukt wird in Ethylacetat gelöst und mit 10% Salzsäure in Ethylacetat versetzt. Der ausfallende Feststoff wird abfiltriert und mit Ethylacetat gewaschen. Ausbeute: 1.3 g (45%; Hydrochlorid); Massenspektroskopie: [M+H]⁺ = 222.

### Synthese der Verbindungen der allgemeinen Formel 1:

### Allgemeine Arbeitsvorschrift 3:

1 mmol Glyoxalaldehyd oder -acetal und 1 mmol Amin werden 30 Minuten in 5 mL Tetrahydrofuran bei Raumtemperatur gerührt. Man kühlt auf 0°C ab und tropft unter Argonatmosphäre 1.5 mL einer 2 molaren Lösung von Lithiumborhydrid in Tetrahydrofuran zu. Es wird 30 min bei Raumtemperatur gerührt, mit 10 mL Dichlormethan und 3 mL Wasser versetzt, eine weitere Stunde gerührt und dann über Kieselgur filtriert, wobei man mit Dichlormethan eluiert. Das Eluat wird vom Lösungsmittel befreit und der Rückstand, falls notwendig, chromatographisch gereinigt.

Der so erhaltene Benzylether wird in Methanol gelöst und mit Palladium auf Kohle (10%ig) als Katalysator bei 2.5 bar und Raumtemperatur hydriert. Anschließend wird der Katalysator abgetrennt und das Rohprodukt mittels Chromatographie (Reverse Phase, Acetonitril/Wasser-Gradient mit 0.1% Trifluoressigsäure) gereinigt oder in Acetonitril auskristallisiert.

### Allgemeine Arbeitsvorschrift 4 (Debenzylierung mit Bortribromid)

1 mmol Glyoxalaldehyd oder -acetal und 1 mmol Amin werden zunächst wie in der allgemeinen Arbeitsvorschrift 3 beschrieben umgesetzt und aufgearbeitet. Anschließend wird der gewonnene Benzylether in 3 mL Dichlormethan gelöst und auf -78°C abgekühlt. Bei dieser Temperatur tropft man langsam 2 mL einer 1 molaren Bortribromid-Lösung in Dichlormethan zu. Die Reaktionsmischung wird auf Raumtemperatur gebracht, weitere 10 Minuten gerührt und dann mit 3 mL Wasser und 10 mL Dichlormethan verdünnt. Die Lösung wird auf eine kurze mit Kieselgur gefüllte Säule gegeben und es wird mit Dichlormethan und Methanol nachgewaschen. Das Eluat wird eingeengt und der Rückstand chromatographiert (Reverse Phase, Acetonitril/Wasser-Gradient mit 0.1 % Trifluoressigsäure).

### Beispiel 1: 5-Hydroxy-8-{1-hydroxy-2-[1-(4-methoxy-benzyl)-cyclopropylamino]-ethyl}-4H-benzo[1,4]oxazin-3-on

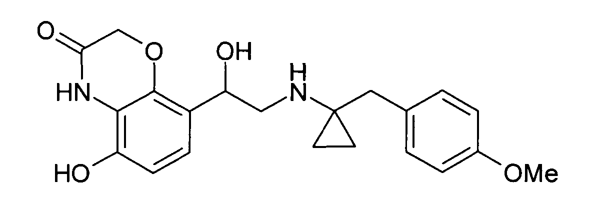

Umsetzung von 329 mg (1 mmol) 5-Benzyloxy-8-(2,2-dihydroxy-acetyl)-4H-benzo[1,4]oxazin-3-on und 177 mg (1 mmol) 1-(4-Methoxy-benzyl)-cyclopropylamin nach der allgemeinen Arbeitsvorschrift 3. Ausbeute: 39 mg (8%, Trifluoracetat); Massenspektroskopie: [M+H]⁺ = 385.

### Beispiel2: 8-[2-(1-Benzyl-cyclopropylamino)-1-hydroxy-ethyl]-5-hydroxy-4H-benzo[1,4]oxazin-3-on

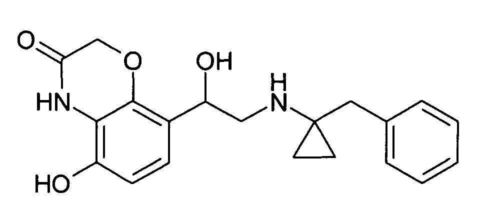

Herstellung nach der allgemeinen Arbeitsvorschrift 3 aus 329 mg (1 mmol) 5-Benzyloxy-8-(2,2-dihydroxy-acetyl)-4H-benzo[1,4]oxazin-3-on und 147 mg (1mmol) 1-Benzyl-cyclopropylamin. Ausbeute: 56 mg (12%, Trifluoracetat); Massenspektroskopie: [M+H]⁺ = 355.

### Beispiel3: 8-{2-[1-(2,6-Dimethyl-benzyl)-cyclopropylamino]-1-hydroxy-ethyl}-5-hydroxy-4H-benzo[1,4]oxazin-3-on

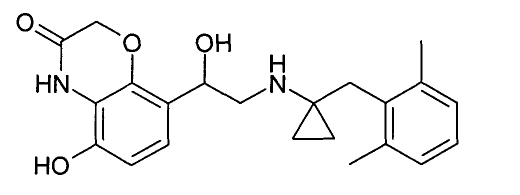

Die Verbindung wird nach der allgemeinen Arbeitsvorschrift 3 aus 329 mg (1 mmol) 5-Benzyloxy-8-(2,2-dihydroxy-acetyl)-4H-benzo[1,4]oxazin-3-on und 175 mg (1 mmol) 1-(2,6-Dimethyl-benzyl)-cyclopropylamin hergestellt. Ausbeute: 52 mg (11%, Trifluoracetat); Massenspektroskopie: [M+H]⁺ = 383.

### Beispiel 4: 8- {2-[1-(3,4-Dimethyl-benzyl)-cyclopropylamino]-1-hydroxy-ethyl}-5-hydroxy-4H-benzo[1,4]oxazin-3-on

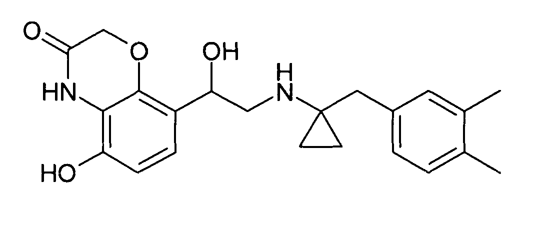

Herstellung nach der allgemeinen Arbeitsvorschrift 3 aus 329 mg (1 mmol) 5-Benzyloxy-8-(2,2-dihydroxy-acetyl)-4H-benzo[1,4]oxazin-3-on und 175 mg (1 mmol) 1-(3,4-Dimethyl-benzyl)-cyclopropylamin. Ausbeute: 233 mg (47%, Trifluoracetat); Massenspektroskopie: [M+H]⁺ = 383.

### Beispiel 5: 5-Hydroxy-8-{1-hydroxy-2-[1-(3-methyl-benzyl)-cyclopropylamino]-ethyl}-4H-benzo[1,4]oxazin-3-on

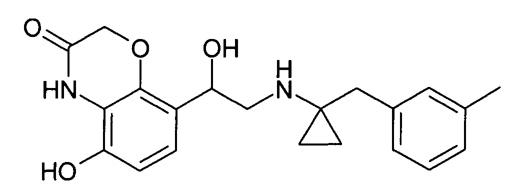

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 161 mg (1 mmol) 1-(3-Methylbenzyl)-cyclopropylamin. Ausbeute: 179 mg (37%, Trifluoracetat); Massenspektroskopie: [M+H]⁺ = 369.

### Beispiel 6: 5-Hydroxy-8-{1-hydroxy-2-[1-(2-trifluormethyl-benzyl)-cyclopropylamino]-ethyl}-4H-benzo[1,4]oxazin-3-on

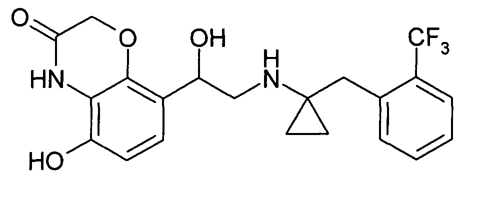

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 215 mg (1 mmol) 1-(2-Trifluormethyl-benzyl)-cyclopropylamin. Ausbeute: 13 mg (2%, Trifluoracetat); Massenspektroskopie: [M+H]⁺ = 423.

### Beispiel 7: 5-Hydroxy-8-(1-hydroxy-2-{1-[4-(1-hydroxy-cyclopropyl)-benzyl] cyclopropylamino}-ethyl)-4H-benzo[1,4]oxazin-3-on

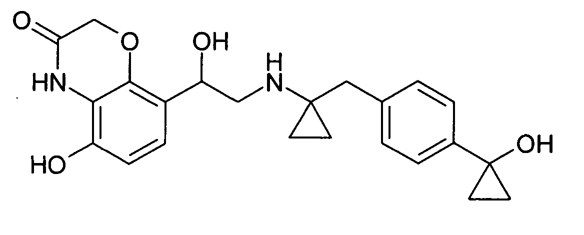

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 203 mg (1 mmol) 1-[4-(1-Amino-cyclopropylmethyl)-phenyl]-cyclopropanol. Ausbeute: 29 mg (6%, Trifluoracetat); Massenspektroskopie: [M+H]⁺ = 411.

### Beispiel 8: 8-{2-[1-(3,5-Difluor-benzyl)-cyclopropylamino]-1-hydroxy-ethyl}-5-hydroxy-4H-benzo[1,4]oxazin-3-on

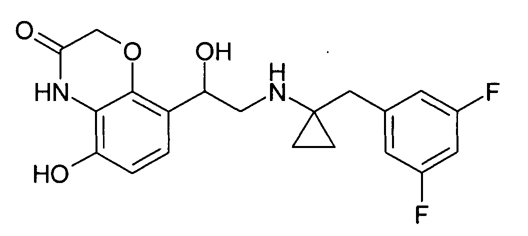

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 183 mg (1 mmol) 1-(3,5-Difluorbenzyl)-cyclopropylamin. Ausbeute: 66 mg (13%, Trifluoracetat); Massenspektroskopie: [M+H]⁺ = 391.

### Beispiel 9: 5-Hydroxy-8-[1-hydroxy-2-(1-phenoxymethyl-cyclopropylamino)-ethyl]-4H-benzo [1,4]oxazin-3-on

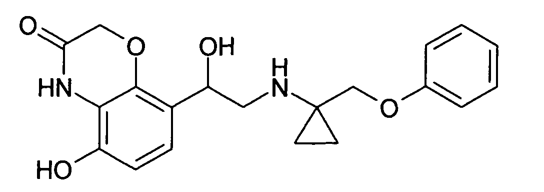

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 163 mg (1 mmol) 1-Phenoxymethyl-cyclopropylamin. Ausbeute: 26 mg (5%, Trifluoracetat); Massenspektroskopie: [M+H]⁺ = 371.

### Beispiel 10: 5-Hydroxy-8-{1-hydroxy-2-[1-(4-trifluormethyl-benzyl)-cyclopropylamino]-ethyl}-4H-benzo[1,4]oxazin-3-on

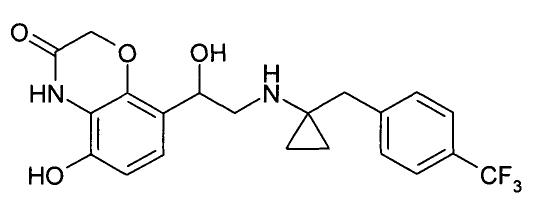

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 215 mg (1 mmol) 1-(4-Trifluormethyl-benzyl)-cyclopropylamin. Ausbeute: 18 mg (3%, Trifluoracetat); Massenspektroskopie: [M+H]⁺ = 423.

### Beispiel 11: 5-Hydroxy-8-{1-hydroxy-2-[1-(2-methoxy-benzyl)-cyclopropylamino]-ethyl}-4H-benzo[1,4]oxazin-3-on

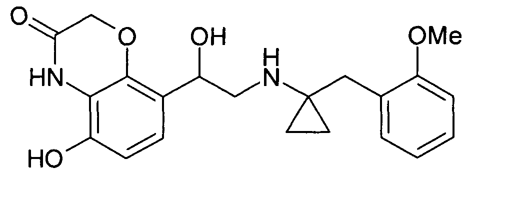

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 177 mg (1 mmol) 1-(2-Methoxybenzyl)-cyclopropylamin. Ausbeute: 100 mg (20%, Trifluoracetat); Massenspektroskopie: [M+H]⁺ = 385.

### Beispiel 12: 5-Hydroxy-8-{1-hydroxy-2-[1-(2-methyl-benzyl)-cyclopropylamino]-ethyl}-4H-benzo[1,4]oxazin-3-on

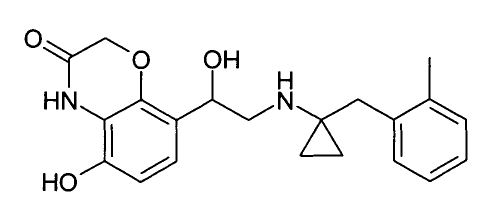

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 161 mg (1 mmol) 1-(2-Methylbenzyl)-cyclopropylamin. Ausbeute: 60 mg (16%, Trifluoracetat); Massenspektroskopie: [M+H]⁺ = 369.

### Beispiel 13: 8-{2-[1-(2,6-Dimethyl-phenoxymethyl)-cyclopropylamino]-1-hydroxy-ethyl}-5-hydroxy-4H-benzo[1,4]oxazin-3-on

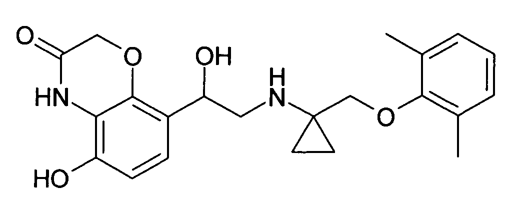

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 191 mg (1 mmol) 1-(2,6-Dimethyl-phenoxymethyl)-cyclopropylamin. Ausbeute: 194 mg (49%, Trifluoracetat); Massenspektroskopie: [M+H]⁺ = 399.

### Beispiel 14: 5-Hydroxy-8-[1-hydroxy-2-(1-naphtalen-2-ylmetyhl-cyclopropylamino)-ethyl]-4H-benzo[1,4]oxazin-3-on

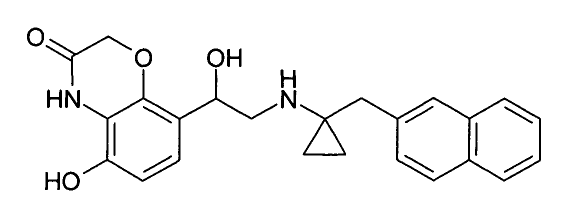

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 197 mg (1 mmol) 1-Naphthalen-2-ylmethyl-cyclopropylamin. Ausbeute: 20 mg (5%, Trifluoracetat); Massenspektroskopie: [M+H]⁺ = 405.

### Beispiel 15: 8-{2-[1-(2,4-Dimethyl-benzyl)-cyclopropylamino]-1-hydroxy-ethyl}-5-hydroxy-4H-benzo[1,4]oxazin-3-on

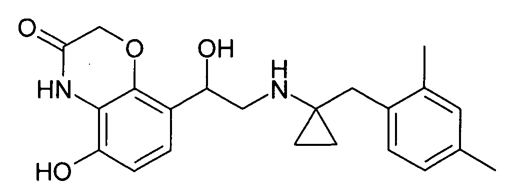

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 175 mg (1 mmol) 1-(2,4-Dimethylbenzyl)-cyclopropylamin. Ausbeute: 176 mg (36%, Trifluoracetat); Massenspektroskopie: [M+H]⁺ = 383.

### Beispiel 16: 5-Hydroxy-8-{1-hydroxy-2-[1-(2,3,5,6-tetramethyl-benzyl)-cyclopropylamino]-ethyl}-4H-benzo[1,4]oxazin-3-on

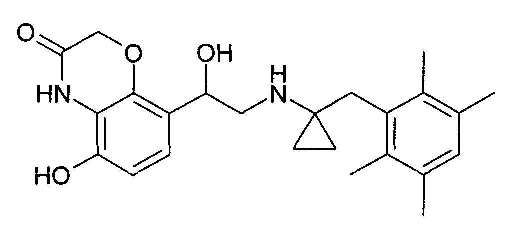

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 203 mg (1 mmol) 1-(2,3,5,6-Tetramethyl-benzyl)-cyclopropylamin. Ausbeute: 93 mg (18%, Trifluoracetat); Massenspektroskopie: [M+H]⁺ = 411.

### Beispiel 17: 5-Hydroxy-8-(1-hydroxy-2-{1-[2-(4-methoxy-phenyl)-ethyl]-cyclopropylamino}-ethyl)-4H-benzo[1,4]oxazin-3-on

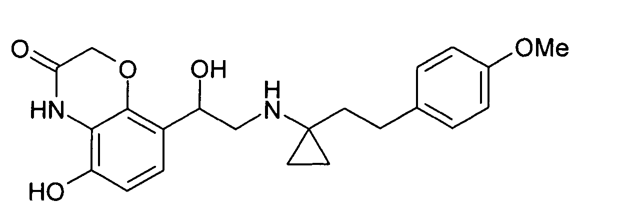

Hergestellt nach der allgemeinen Arbeitsvorschrift 3 aus 329 mg (1 mmol) 5-Benzyloxy-8 (2,2-dihydroxy-acetyl)-4H-benzo[1,4]oxazin-3-on und 191 mg (1 mmol) 1-[2-(4-Methoxy phenyl)-ethyl]-cyclopropylamin. Ausbeute: 55 mg (11%, Trifluoracetat); Massenspektroskopie: [M+H]⁺ = 399.

### Beispiel 18: 8-{2-[1-(2,6-Difluor-benzyl)-cyclopropylamino]-1-hydroxy-ethyl}-5-hydroxy-4H-benzo[1,4]oxazin-3-on

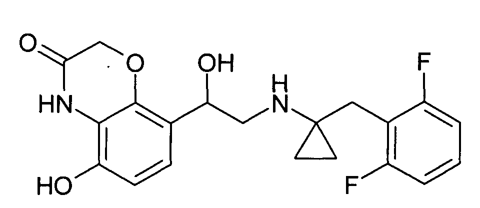

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 183 mg (1 mmol) 1-(2,6-Difluorbenzyl)-cyclopropylamin. Ausbeute: 108 mg (21 %, Trifluoracetat); Massenspektroskopie: [M+H]⁺ = 391.

### Beispiel 19: 8-{2-[1-(4-Amino-benzyl)-cyclopropylamino]-1-hydroxy-ethyl}-5-hydroxy 4H-benzo[1,4]oxazin-3-on

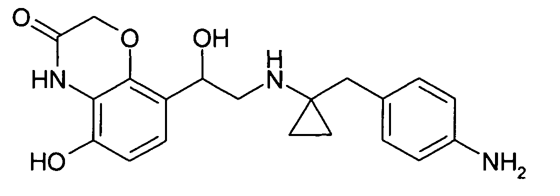

Hergestellt nach der allgemeinen Arbeitsvorschrift 3 aus 329 mg (1 mmol) 5-Benzyloxy-8-(2,2-dihydroxy-acetyl)-4H-benzo[1,4]oxazin-3-on und 342 mg (1 mmol) [4-(1-Amino-cyclopropylmethyl)-phenyl]-dibenzyl-amin. Ausbeute: 49 mg (10%, Trifluoracetat); Massenspektroskopie: [M+H]⁺ = 370.

### Beispiel 20: 5-Hydroxy-8-{1-hydroxy-2-[1-(4-hydroxy-benzyl)-cyclopropylamino]-ethyl}-4H-benzo[1,4]oxazin-3-on

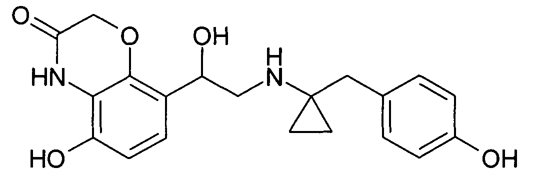

Herstellung nach der allgemeinen Arbeitsvorschrift 3 aus 329 mg (1 mmol) 5-Benzyloxy-8-(2,2-dihydroxy-acetyl)-4H-benzo[1,4]oxazin-3-on und 253 mg (1 mmol) 1-(4-Benzyloxy-benzyl)-cyclopropylamin. Ausbeute: 20 mg (4%, Trifluoracetat); Massenspektroskopie: [M+H]⁺ = 371.

### Beispiel 21: 8-{2-[1-(3,4-Dimethoxy-benzyl)-cyclopropylamino]-1-hydroxy-ethyl}-5-hydroxy-4H-benzo[1,4]oxazin-3-on

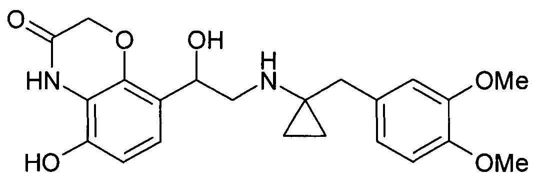

Herstellung nach der allgemeinen Arbeitsvorschrift 3 aus 329 mg (1 mmol) 5-Benzyloxy-8-(2,2-dihydroxy-acetyl)-4H-benzo[1,4]oxazin-3-on und 207 mg (1 mmol) 1-(3,4-Dimethoxy-benzyl)-cyclopropylamin. Ausbeute: 15 mg (3%, Trifluoracetat); Massenspektroskopie: [M+H]⁺ = 415.

### Beispiel 22: 8-{2-[1-(4-Ethoxy-benzyl)-cyclopropylamino]-1-hydroxy-ethyl}-5-hydroxy-4H-benzo[1,4]oxazin-3-on

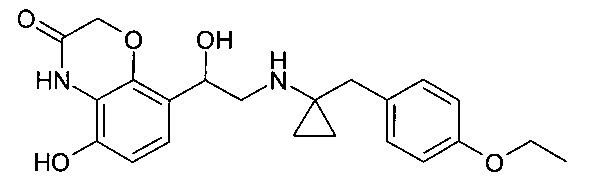

Herstellung nach der allgemeinen Arbeitsvorschrift 3 aus 329 mg (1 mmol) 5-Benzyloxy-8-(2,2-dihydroxy-acetyl)-4H-benzo[1,4]oxazin-3-on und 191 mg (1 mmol) 1-(4-Ethoxybenzyl)-cyclopropylamin. Ausbeute: 17 mg (3%, Trifluoracetat); Massenspektroskopie: [M+H]⁺ = 399.

### Beispiel 23: 8-{2-[1-(4-Butoxy-benzyl)-cyclopropylamino]-1-hydroxy-ethyl}-5-hydroxy-4H-benzo[1,4]oxazin-3-on

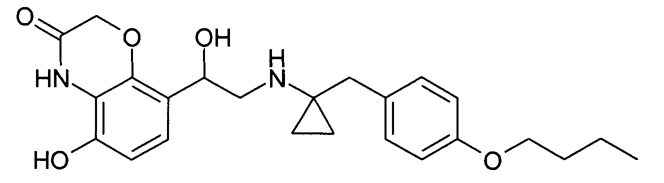

Herstellung nach der allgemeinen Arbeitsvorschrift 3 aus 329 mg (1 mmol) 5-Benzyloxy-8-(2,2-dihydroxy-acetyl)-4H-benzo[1,4]oxazin-3-on und 219 mg (1 mmol) 1-(4-Butoxy-benzyl)-cyclopropylamin. Ausbeute: 107 mg (20%, Trifluoracetat); Massenspektroskopie: [M+H]⁺ = 427.

### Beispiel 24: 5-Hydroxy-8-{1-hydroxy-2-[1-(2,4,6-trimethyl-benzyl)-cyclopropylamino]-ethyl}-4H-benzo[1,4]oxazin-3-on

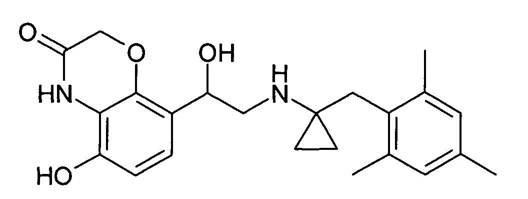

Herstellung nach der allgemeinen Arbeitsvorschrift 3 aus 329 mg (1 mmol) 5-Benzyloxy-8-(2,2-dihydroxy-acetyl)-4H-benzo[1,4]oxazin-3-on und 189 mg (1 mmol) 1-(2,4,6-Trimethyl-benzyl)-cyclopropylamin. Ausbeute: 26 mg (5%, Trifluoracetat); Massenspektroskopie: [M+H]⁺ = 397.

### Beispiel 25: 5-Hydroxy-8-{1-hydroxy-2-[(1-phenyl-cyclopropylmethyl)-amino]-ethyl}-4H-benzo[1,4]oxazin-3-on

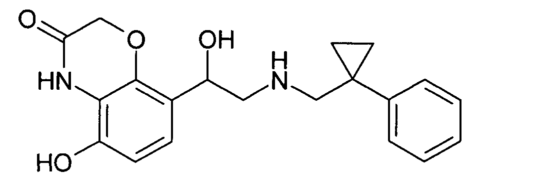

Hergestellt aus 329 mg (1 mmol) 5-Benzyloxy-8-(2,2-dihydroxy-acetyl)-4H-benzo[1,4]oxazin-3-on und 147 mg (1 mmol) C-(1-Phenyl-cyclopropyl)-methylamin. Abschließende Reinigung mittels Chromatographie (Kieselgel; Dichlormethan/Methanol-Gradient). Ausbeute: 30 mg (8%); Massenspektroskopie: [M+H]⁺ = 355.

### Beispiel 26: 5-Hydroxy-8-{1-hydroxy-2-[1-(2-naphthalen-2-yl-ethyl)-cyclopropylamino]-ethyl}-4H-benzo[1,4]oxazin-3-on

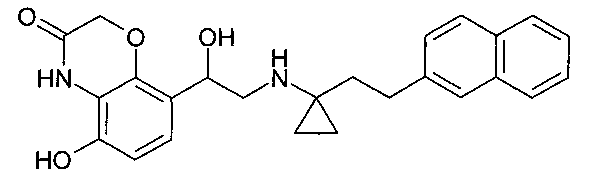

Hergestellt aus 5-Benzyloxy-8-(2,2-dihydroxy-acetyl)-4H-benzo[1,4]oxazin-3-on und 1-(2-Naphthalen-2-yl-ethyl)-cyclopropylamin. Massenspektroskopie: [M+H]⁺ = 419.

### Allgemeine Arbeitsvorschrift 5:

1 mmol 2-Benzyloxy-5-(2-ethoxy-2-hydroxy-acetyl)-benzoesäuremethylester und 1 mmol Amin werden 30 Minuten in 5 mL Tetrahydrofuran bei Raumtemperatur gerührt. Man kühlt auf 0°C ab und tropft unter Argonatmosphäre 1.5 mL einer 2 molaren Lösung von Lithiumborhydrid in Tetrahydrofuran zu. Nach 30 Minuten Rühren bei Raumtemperatur werden weitere 1.5 mL der 2 molaren Lösung von Lithiumborhydrid in Tetrahydrofuran zugegeben und die Reaktionslösung wird 4 Stunden auf 50°C erwärmt. Es wird mit 10 mL Dichlormethan und 3 mL Wasser versetzt, eine weitere Stunde gerührt und dann über Kieselgur filtriert, wobei man mit Dichlormethan eluiert. Das Eluat wird eingeengt und der Rückstand wird in Methanol gelöst und in Gegenwart von Palladium auf Kohle (10%ig) bei 2.5 bar und Raumtemperatur hydriert. Anschließend wird der Katalysator abgetrennt und das Rohprodukt chromatographisch (Reverse Phase, Acetonitril/Wasser-Gradient mit 0.1 % Trifluoressigsäure) gereinigt.

### Beispiel 27: 2-Hydroxymethyl-4-{1-hydroxy-2-[(1-phenyl-cyclopropylmethyl)-amino]-ethyl}-phenol

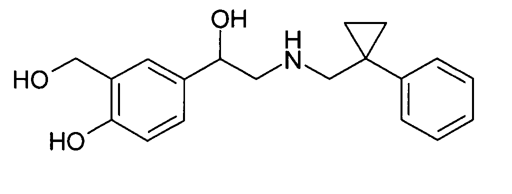

Herstellung aus 344 mg (1 mmol) 2-Benzyloxy-5-(2-ethoxy-2-hydroxy-acetyl)-benzoesäuremethylester und 147 mg (1 mmol) 1-Phenyl-cyclopropyl-methylamin. Abweichend von der allgemeinen Arbeitsvorschrift 5 wird die Zielverbindung durch Chromatographie an einer Kieselgelsäule (Dichormethan/Methanol-Gradient) gereinigt. Ausbeute: 162 mg (52%); Massenspektroskopie: [M+H]⁺ = 314.

### Beispiel 28: 4-{1-Hydroxy-2-[1-(4-methoxy-benzyl)-cyclopropylamino]-ethyl}-2-hydroxymethyl-phenol

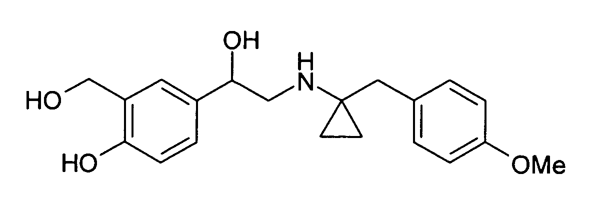

Herstellung nach der allgemeinen Arbeitsvorschrift 5 aus 344 mg (1 mmol) 2-Benzyloxy-5-(2-ethoxy-2-hydroxy-acetyl)-benzoesäuremethylester und 177 mg (1 mmol) 1-(4-Methoxy-benzyl)-cyclopropylamin. Ausbeute: 123 mg (27%, Trifluoracetat); Massenspektroskopie: [M+H]⁺ = 344.

### Beispiel 29: 4-[2-(1-Benzyl-cyclopropylamino)-1-hydroxy-ethyl]-2-hydroxymethyl-phenol

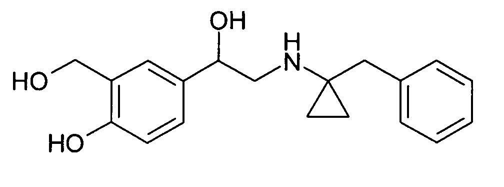

Herstellung nach der allgemeinen Arbeitsvorschrift 5 aus 344 mg (1 mmol) 2-Benzyloxy-5-(2-ethoxy-2-hydroxy-acetyl)-benzoesäuremethylester und 147 mg (1 mmol) 1-Benzyl-cyclopropylamin. Ausbeute: 184 mg (43%, Trifluoracetat); Massenspektroskopie: [M+H]⁺ = 314.

### Beispiel 30: 4-{2-[1-(2,6-Dimethyl-benzyl)-cyclopropylamino]-1-hydroxy-ethyl}-2-hydroxymethyl-phenol

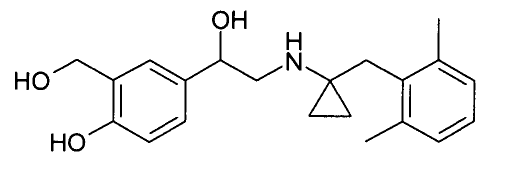

Herstellung nach der allgemeinen Arbeitsvorschrift 5 aus 344 mg (1 mmol) 2-Benzyloxy-5-(2-ethoxy-2-hydroxy-acetyl)-benzoesäuremethylester und 175 mg (1 mmol) 1-(2,6-Dimethyl-benzyl)-cyclopropylamin. Ausbeute: 64 mg (14%, Trifluoracetat);
Massenspektroskopie: [M+H]⁺ = 342.

### Beispiel 31: 6-Hydroxy-8-{1-hydroxy-2-[1-(4-methoxy-benzyl)-cyclopropylamino]-ethyl}-4H-benzo[1,4]oxazin-3-on

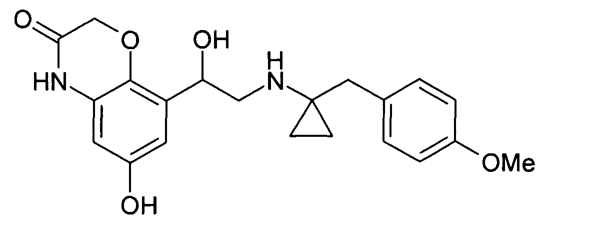

Hergestellt aus 357 mg (1 mmol) 6-Benzyloxy-8-(2-ethoxy-2-hydroxy-acetyl)-4H-benzo[1,4]oxazin-3-on und 177 mg (1 mmol) 1-(4-Methoxyoxy-benzyl)-cyclopropylamin. Ausbeute: 109 mg (22%, Trifluoracetat); Massenspektroskopie: [M+H]⁺ = 385.

### Beispiel 32: 6-Hydroxy-8-{1-hydroxy-2-[1-(4-hydroxy-benzyl)-cyclopropylamino]-ethyl}-4H-benzo[1,4]oxazin-3-on

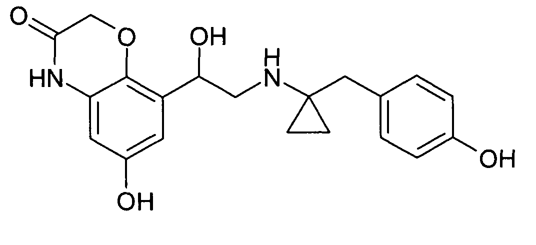

Herstellung nach der allgemeinen Arbeitsvorschrift 4 aus 357 mg (1 mmol) 6-Benzyloxy-8-(2-ethoxy-2-hydroxy-acetyl)-4H-benzo[1,4]oxazin-3-on und 253 mg (1 mmol) 1-(4-Benzyloxy-benzyl)-cyclopropylamin. Ausbeute: 47 mg (10%, Trifluoracetat); Massenspektroskopie: [M+H]⁺ = 371.

### Beispiel 3 3 : 6-Hydroxy-8-{1-hydroxy-2-[(1-phenyl-cyclopropylmethyl)-amino]-ethyl}-4H-benzo[1,4]oxazin-3-on

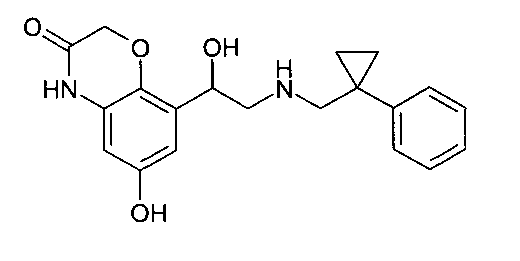

Hergestellt aus 357 mg (1 mmol) 6-Benzyloxy-8-(2-ethoxy-2-hydroxy-acetyl)-4H-benzo[1,4]oxazin-3-on und 147 mg (1 mmol) (1-Phenyl-cyclopropyl)-methylamin. Ausbeute: 138 mg (39%); Massenspektroskopie: [M+H]⁺ = 355.

### Beispiel 34: 8-{2-[1-(2,6-Dimethyl-benzyl)-cyclopropylamino]-1-hydroxy-ethyl}-6-hydroxy-4H-benzo[1,4]oxazin-3-on

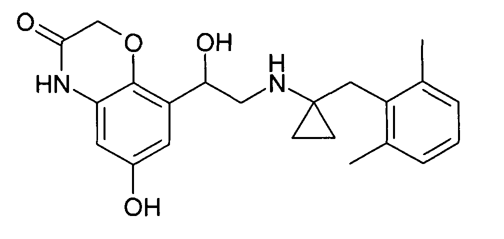

Erhalten aus 357 mg (1 mmol) 6-Benzyloxy-8-(2-ethoxy-2-hydroxy-acetyl)-4H-benzo[1,4]oxazin-3-on und 175 mg (1 mmol) 1-(2,6-Dimethyl-benzyl)-cyclopropylamin. Ausbeute: 93 mg (19%, Trifluoracetat); Massenspektroskopie: [M+H]⁺= 383.

### Beispiel 35: 8-{2-[1-(3,5-Dimethyl-benzyl)-cyclopropylamino]-1-hydroxy-ethyl}-6-hydroxy-4H-benzo[1,4]oxazin-3-on

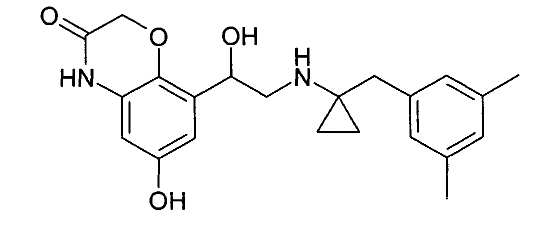

Hergestellt aus 357 mg (1 mmol) 6-Benzyloxy-8-(2-ethoxy-2-hydroxy-acetyl)-4H-benzo[1,4]oxazin-3-on und 175 mg (1 mmol) 1-(3,5-Dimethyl-benzyl)-cyclopropylamin nach der allgemeinen Arbeitsvorschrift 2. Ausbeute: 155 mg (31%, Trifluoracetat); Massenspektroskopie: [M+H]⁺ = 383.

### Beispiel 36: 6-Hydroxy-8-{1-hydroxy-2-[1-(4-trifluormethoxy-benzyl)-cyclopropylamino]-ethyl}-4H-benzo[1,4]oxazin-3-on

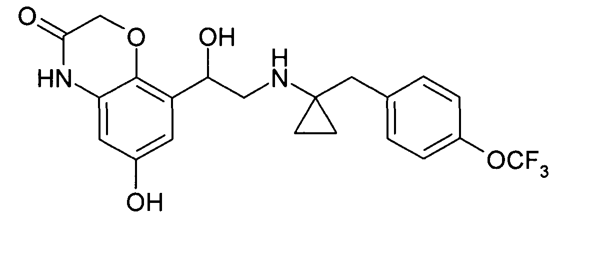

Hergestellt aus 357 mg (1 mmol) 6-Benzyloxy-8-(2-ethoxy-2-hydroxy-acetyl)-4H-benzo[1,4]oxazin-3-on und 231 mg (1 mmol) 1-(4-Trifluormethoxy-benzyl)-cyclopropylamin nach der allgemeinen Arbeitsvorschrift 3. Ausbeute: 148 mg (27%, Trifluoracetat); Massenspektroskopie: [M+H]⁺ = 439.

### Beispiel 37: 8-{2-[1-(2,4-Dichlor-benzyl)-cyclopropylamino]-1-hydroxy-ethyl}-6-hydroxy-4H-benzo[1,4]oxazin-3-on

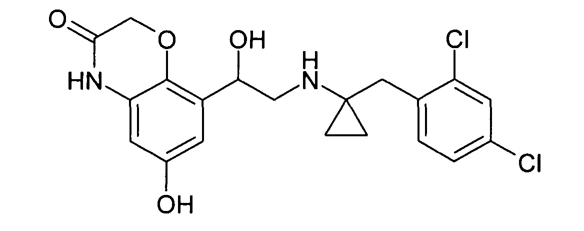

Aus der Umsetzung von 357 mg (1 mmol) 6-Benzyloxy-8-(2-ethoxy-2-hydroxy-acetyl)-4H-benzo[1,4]oxazin-3-on und 216 mg (1 mmol) 1-(2,4-Dichlor-benzyl)-cyclopropylamin werden 458 mg (0.73 mmol) Benzylether als Trifluoracetat erhalten. Die anschließende Debenzylierung erfolgt nach der allgemeinen Arbeitsvorschrift 4 mit Bortribromid.
Ausbeute: 158 mg (29% über 2 Stufen; Trifluoracetat); Massenspektroskopie: [M+H]⁺ = 423/5/7.

### Beispiel 38: 8-{2-[1-(4-Chlor-phenoxymethyl)-cyclopropylamino]-1-hydroxy-ethyl}-6-hydroxy-4H-benzo[1,4]oxazin-3-on

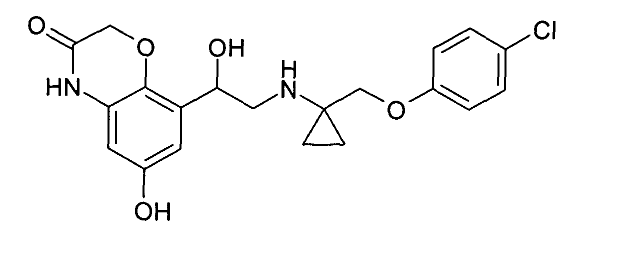

Die Umsetzung von 357 mg (1 mmol) 6-Benzyloxy-8-(2-ethoxy-2-hydroxy-acetyl)-4H-benzo[1,4]oxazin-3-on und 198 mg (1 mmol) 1-(4-Chlor-phenoxymethyl)-cyclopropylamin liefert 259 mg (0.4 mmol) Benzylether (Trifluoracetat). Dieser wird nach der allgemeinen Arbeitsvorschrift 4 mit Bortribromid debenzyliert. Ausbeute: 99 mg (19% über 2 Stufen; Trifluoracetat); Massenspektroskopie: [M+H]⁺ = 405/7.

Die Beispiele 39 bis 42 werden nach der allgemeinen Arbeitsvorschrift 3 hergestellt mit dem Unterschied, dass eine Benzylschutzgruppenabspaltung nicht erforderlich ist. Die angegebenen Ausbeuten beziehen sich jeweils auf die Umsetzung von 1 mmol 1-(4-Amino-3,5-dichlor-phenyl)-2,2-dihydroxy-ethanon und 1 mmol Amin.

### Beispiel 39: 1-(4-Amino-3,5-dichlor-phenyl)-2-[(1-phenyl-cyclopropylmethyl)-amino}-ethanol

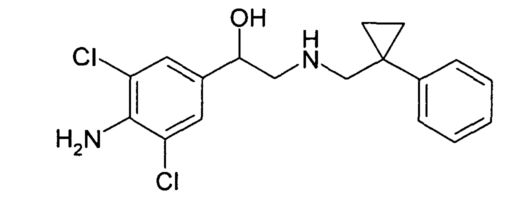

Ausbeute: 63 mg (14%, Trifluoracetat); Massenspektroskopie: [M+H]⁺ = 351/353/355.

### Beispiel 40: 1-(4-Amino-3,5-dichlor-phenyl)-2-[1-(2,6-dimethyl-benzyl)-cyclopropylamino]-ethanol

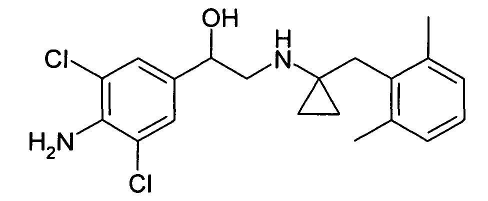

Ausbeute: 101 mg (21%, Trifluoracetat); Massenspektroskopie: [M+H]⁺ = 379/381/383.

### Beispiel 41: 1-(4-Amino-3,5-dichlor-phenyl)-2-[1-(4-methoxy-benzyl)-cyclopropylamin]-ethanol

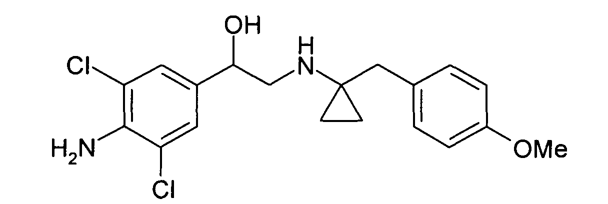

Ausbeute: 167 mg (34%, Trifluoracetat); Massenspektroskopie: [M+H]⁺ = 381/383/385.

### Beispiel 42: 1-(4-Amino-3,5-dichlor-phenyl)-2-(1-benzyl-cyclopropylamino)-ethanol

Ausbeute: 271 mg (58%, Trifluoracetat); Massenspektroskopie: [M+H]⁺ = 351/353/355.

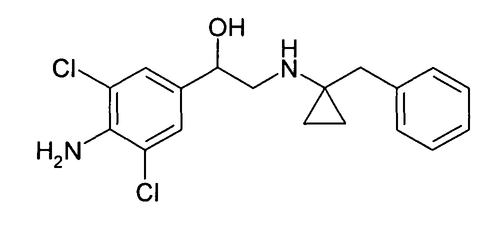

### Beispiel 43: 8-Hydroxy-5-{1-hydroxy-2-[1-(4-methoxy-benzyl)-cyclopropylamino]-ethyl}-1H-chinolin-2-on

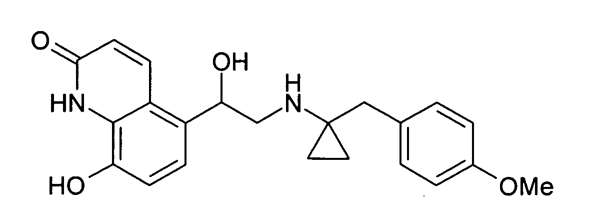

### a) 8-Benzyloxy-5-{1-hydroxy-2-[1-(4-methoxy-benzyl)-cyclopropylamino]-ethyl}-1H-chinolin-2-on

587 mg (2.0 mmol) 8-Benzyloxy-5-oxiranyl-1H-chinolin-2-on und 480 mg (2.7 mmol) 1-(4-Methoxy-benzyl)-cyclopropylamin werden in 10 mL n-Butanol über 10 Stunden unter Rückfluß erwärmt. Anschließend wird das Lösungsmittel abdestilliert und der Rückstand chromatographisch (Reverse Phase, Wasser/Acetonitril-Gradient) gereinigt. Ausbeute: 259 mg (27%); Massenspektroskopie: [M+H]⁺ = 471.

### b) 8-Hydroxy-5-{1-hydroxy-2-[1-(4-methoxy-benzyl)-cyclopropylamino]-ethyl}-1H-chinolin-2-on

258 mg (0.51 mmol) 8-Benzyloxy-5-{1-hydroxy-2-[1-(4-methoxy-benzyl)-cyclopropylamin]-ethyl}-1H-chinolin-2-on in 10 mL Methanol werden mit Palladium auf Kohle als Katalysator bei 3 bar hydriert. Anschließend wird der Katalysator abgetrennt, das Lösungsmittel abdestilliert und der Rückstand mittels Chromatographie (Reverse Phase, Wasser/Acetonitril-Gradient) gereinigt. Ausbeute: 12 mg (6%); Massenspektroskopie: [M+H]⁺ = 381.

### Beispiel 44: 8-Hydroxy-5-{1-hydroxy-2-[1-(4-trifluormethyl-benzyl)-cyclopropylamino]-ethyl}-1H-chinolin-2-on

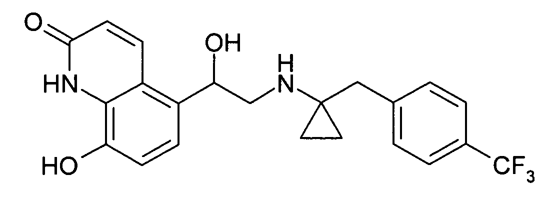

### a) 8-Benzyloxy-5-{1-hydroxy-2-[1-(4-trifluormethyl-benzyl)-cyclopropylamino]-ethyl}-1H-chinolin-2-on

Umsetzung von 587 mg (2.0 mmol) 8-Benzyloxy-5-oxiranyl-1H-chinolin-2-on und 710 mg (3.3 mmol) 1-(4-Trifluormethyl-benzyl)-cyclopropylamin erfolgt in Analogie zu Beispiel 40a). Ausbeute: 322 mg (32%); Massenspektroskopie: [M+H]⁺ = 509.

### b) 8-Hydroxy-5-{1-hydroxy-2-[1-(4-trifluormethyl-benzyl)-cyclopropylamino]-ethyl}-1H-chinolin-2-on

322 mg (0.63 mmol) 8-Benzyloxy-5-{1-hydroxy-2-[1-(4-trifluormethyl-benzyl)-cyclopropylamino]-ethyl}-1H-chinolin-2-on werden in 10 mL Methanol gelöst und in Gegenwart von Palladium auf Kohle bei 3 bar hydriert. Durch Zugabe von Methanol wird ausgefallenes Produkt in Lösung gebracht. Der Katalysator wird abgetrennt und das Filtrat eingeengt. Der dabei ausfallende Feststoff wird abfiltriert und zur weiteren Reinigung in Diethylether suspendiert. Ausbeute: 161 mg (61%); Massenspektroskopie: [M+H]⁺ = 419.

### Allgemeine Arbeitsvorschrift 6

7 mmol des als Hydrochlorid vorliegenden Amins werden mit 1.7 mmol Triethylamin für 30 Minuten in 6 mL Tetrahydrofuran gerührt. Dann werden 1.7 mmol Glyoxylaldehyd oder
-acetal hinzugegeben und man läßt weitere 1.5 Stunden rühren. Die Reaktionsmischung wird auf 10°C abgekühlt, mit 150 mg (6.9 mmol) Lithiumborhydrid versetzt, 30 Minuten gerührt und dann mit Wasser versetzt. Anschließend extrahiert man mit Dichlormethan und trocknet die organische Phase mit Natriumsulfat. Das Lösungsmittel wird abdestilliert und der Rückstand in Ethylacetat gelöst und mit 10% Salzsäure in Ethylacetat versetzt. Der ausfallende Feststoff wird abfiltriert, in Methanol vorgelegt und mit Palladium auf Kohle bei Normaldruck hydriert. Anschließend wird der Katalysator abgetrennt und das Filtrat vom Lösungsmittel befreit. Durch Verrühren des Rückstands in Ethylacetat oder Kristallisation aus dem gleichen Lösungsmittel wird das Produkt in Form eines farblosen Feststoffs erhalten.

### Beispiel 45 : 6-Hydroxy-8-[1-hydroxy-2-(1-phenethyl-cyclohexylamino)-ethyl]-4H-benzo[1,4]oxazin-3-one

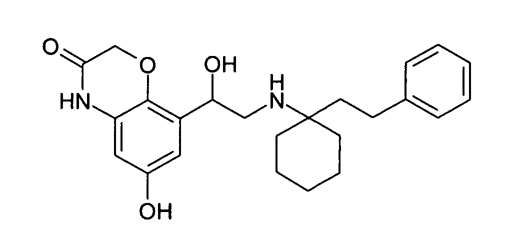

Hergestellt nach der allgemeinen Arbeitsvorschrift 6 aus 607 mg (1.70 mmol) 6-Benzyloxy-8-(2-ethoxy-2-hydroxy-acetyl)-4H-benzo[1,4]oxazin-3-on und 408 mg (1.70 mmol) 1-Phenethylcyclohexylamin Hydrochlorid. Ausbeute: 289 mg (38%, Hydrochlorid), Massenspektroskopie: [M+H]⁺ = 411.

### Beispiel 46: 8-[2-(1-Benzyl-cyclopentylamino)-1-hydroxy-ethyl]-6-hydroxy-4H-benzo[1,4]oxazin-3-on

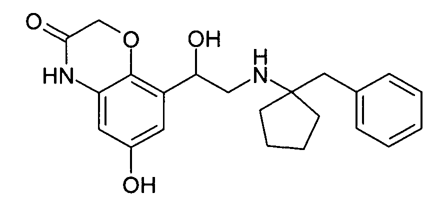

Hergestellt nach der allgemeinen Arbeitsvorschrift 6 aus 607 mg (1.70 mmol) 6-Benzyloxy-8-(2-ethoxy-2-hydroxy-acetyl)-4H-benzo[1,4]oxazin-3-on und 360 mg (1.70 mmol) 1-Benzylcyclopentylamin Hydrochlorid. Ausbeute: 421 mg (59%, Hydrochlorid), Massenspektroskopie: [M+H]⁺ = 383.

### Beispiel 47: 8-{2-[1-(4-Fluor-benzyl)-cyclopentylamino]-1-hydroxy-ethyl}-6-hydroxy-4H-benzo[1,4]oxazin-3-on

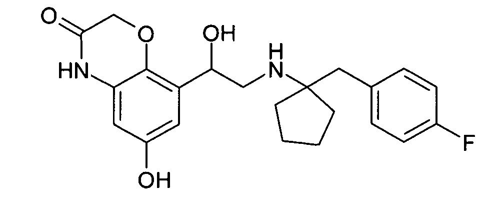

Hergestellt nach der allgemeinen Arbeitsvorschrift 6 aus 607 mg (1.70 mmol) 6-Benzyloxy-8-(2-ethoxy-2-hydroxy-acetyl)-4H-benzo[1,4]oxazin-3-on und 414 mg (1.70 mmol) 1-(4-Fluor-benzyl)-cyclopentylamin Hydrochlorid. Ausbeute: 447 mg (60%, Hydrochlorid), Massenspektroskopie: [M+H]⁺ = 401.

### Beispiel 48: 6-Hydroxy-8-(1-hydroxy-2-{1-[2-(4-methoxy-phenyl)-ethyl]-cyclohexylamino}-ethyl)-4H-benzo[1,4]oxazin-3-on

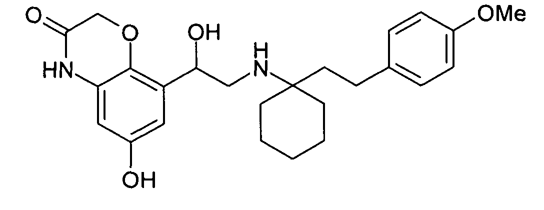

Hergestellt nach der allgemeinen Arbeitsvorschrift 6 aus 607 mg (1.70 mmol) 6-Benzyloxy-8-(2-ethoxy-2-hydroxy-acetyl)-4H-benzo[1,4]oxazin-3-on und 458 mg (1.70 mmol) 1-[2-(4-Methoxy-phenyl)-ethyl]-cyclohexylamin Hydrochlorid. Ausbeute: 559 mg (65%, Hydrochlorid), Massenspektroskopie: [M+H]⁺ = 441.

### Beispiel 49: 6-Hydroxy-8-{1-hydroxy-2-[1-(4-methoxy-benzyl)-cyclopentylamino]-ethyl}-4H-benzo[1,4]oxazin-3-on

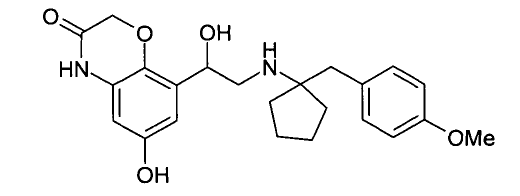

Hergestellt aus 607 mg (1.70 mmol) 6-Benzyloxy-8-(2-ethoxy-2-hydroxy-acetyl)-4H-benzo[1,4]oxazin-3-on und 411 mg (1.70 mmol) 1-(4-Methoxy-benzyl)-cyclopentylamin Hydrochlorid nach der allgemeinen Arbeitsvorschrift 6. Ausbeute: 428 mg (56%, Hydrochlorid). Massenspektroskopie: [M+H]⁺ = 413.

### Beispiel 50: 6-Hydroxy-8-{1-hydroxy-2-[1-(4-methoxy-benzyl)-cyclohexylamino]-ethyl} -4H-benzo[1,4]oxazin-3-on

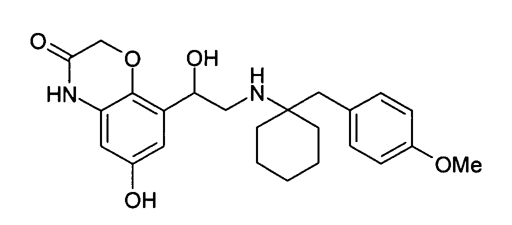

Erhalten aus 607 mg (1.70 mmol) 6-Benzyloxy-8-(2-ethoxy-2-hydroxy-acetyl)-4H-benzo[1,4]oxazin-3-on und 435 mg (1.70 mmol) 1-(4-Methoxy-benzyl)-cyclohexylamin Hydrochlorid nach der allgemeinen Arbeitsvorschrift 6. Ausbeute: 521 mg (66%, Hydrochlorid). Massenspektroskopie: [M+H]⁺ = 427.

### Beispiel 51: 8-(2-{1-[2-(4-Fluor-phenyl)-ethyl]-cyclohexylamino}-1-hydroxy-ethyl)-6-hydroxy-4H-benzo[1,4]oxazin-3-on

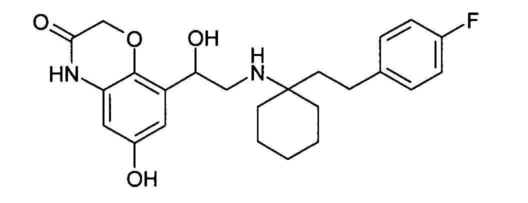

Hergestellt nach der allgemeinen Arbeitsvorschrift 6 aus 607 mg (1.70 mmol) 6-Benzyloxy-8-(2-ethoxy-2-hydroxy-acetyl)-4H-benzo[1,4]oxazin-3-on und 438 mg (1.70 mmol) 1[2-(4-fluorophenyl)-ethyl]-cyclohexylamin Hydrochlorid. Ausbeute: 507 mg (64%, Hydrochlorid). Massenspektroskopie: [M+H]⁺ = 429.

### Beispiel 52: 5-Hydroxy-8-[1-hydroxy-2-(1-phenethyl-cyclohexylamino)-ethyl]-4H-benzo[1,4]oxazin-3-on

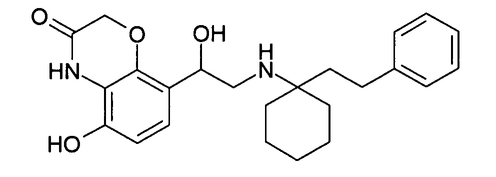

Erhalten aus 560 mg (1.70 mmol) 5-Benzyloxy-8-(2,2-dihydroxy-acetyl)-4H-benzo[1,4]oxazin-3-on und 408 mg (1.70 mmol) 1-Phenethylcyclohexylamin Hydrochlorid nach der allgemeinen Arbeitsvorschrift 6. Ausbeute: 320 mg (42%, Hydrochlorid). Massenspektroskopie: [M+H]⁺ = 411.

### Beispiel 53: 8-[2-(1-Benzyl-cyclopentylamino)-1-hydroxy-ethyl]-5-hydroxy-4H-benzo [1,4]oxazin-3-on

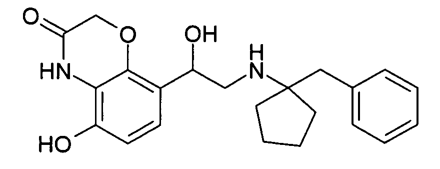

Hergestellt aus 560 mg (1.70 mmol) 5-Benzyloxy-8-(2,2-dihydroxy-acetyl)-4H-benzo[1,4]oxazin-3-on und 360 mg (1.70 mmol) 1-Benzylcyclopentylamin Hydrochlorid nach der allgemeinen Arbeitsvorschrift 6. Ausbeute: 328 mg (46%, Hydrochlorid). Massenspektroskopie: [M+H]⁺ = 383.

### Beispiel 54: 8-{2-[1-(4-Fluor-benzyl)-cyclopentylamino]-1-hydroxy-ethyl}-5-hydroxy-4H-benzo[1,4]oxazin-3-on

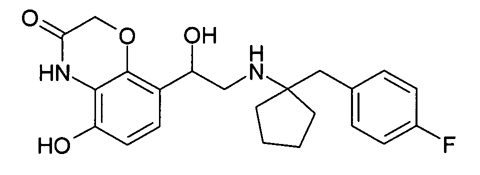

Hergestellt aus 560 mg (1.70 mmol) 5-Benzyloxy-8-(2,2-dihydroxy-acetyl)-4H-benzo[1,4]oxazin-3-on und 414 mg (1.70 mmol) 1-(4-Fluor-benzyl)-cyclopentylamin Hydrochlorid nach der allgemeinen Arbeitsvorschrift 6. Ausbeute: 454 mg (61%, Hydrochlorid). Massenspektroskopie: [M+H]⁺ = 401.

### Beispiel 55: 5-Hydroxy-8-(1-hydroxy-2-{1-[2-(4-methoxy-phenyl)-ethyl]-cyclohexylamino}-ethyl)-4H-benzo[1,4]oxazin-3-on

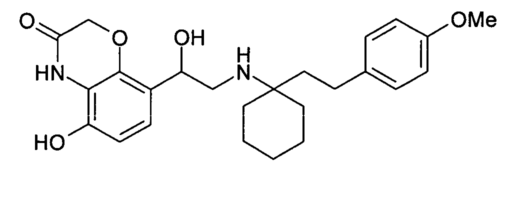

Erhalten aus 560 mg (1.70 mmol) 5-Benzyloxy-8-(2,2-dihydroxy-acetyl)-4H-benzo[1,4]oxazin-3-on und 458 mg (1.70 mmol) 1-[2-(4-Methoxy-phenyl)-ethyl]-cyclohexylamin Hydrochlorid nach der allgemeinen Arbeitsvorschrift 6. Ausbeute: 544 mg (67%, Hydrochlorid). Massenspektroskopie: [M+H]⁺ = 441.

### Beispiel 56: 5-Hydroxy-8-{1-hydroxy-2-[1-(4-methoxy-benzyl)-cyclopentylamino]-ethyl}-4H-benzo[1,4]oxazin-3-on

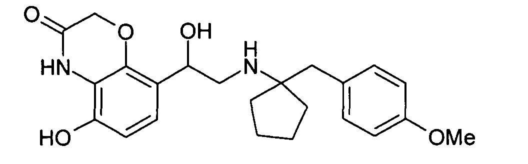

Hergestellt aus 560 mg (1.70 mmol) of 5-Benzyloxy-8-(2,2-dihydroxy-acetyl)-4H-benzo[1,4]oxazin-3-on und 411 mg (1.70 mmol) 1-(4-Methoxy-benzyl)-cyclopentylamin Hydrochlorid nach der allgemeinen Arbeitsvorschrift 6. Ausbeute: 467 mg (61 %, Hydrochlorid). Massenspektroskopie: [M+H]⁺ = 413.

### Beispiel 57: 5-Hydroxy-8-{1-hydroxy-2-[1-(4-methoxy-benzyl)-cyclohexylamino]-ethyl} -4H-benzo[1,4]oxazin-3-on

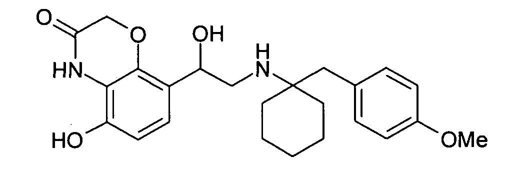

Hergestellt aus 560 mg (1.70 mmol) 5-Benzyloxy-8-(2,2-dihydroxy-acetyl)-4H-benzo[1,4]oxazin-3-on und 435 mg (1.70 mmol) 1-(4-Methoxy-benzyl)-cyclohexylamin Hydrochlorid nach der allgemeinen Arbeitsvorschrift 6. Ausbeute: 426 mg (54%, Hydrochlorid). Massenspektroskopie: [M+H]⁺ = 427.

### Beispiel 58: 8-(2-{1-[2-(4-Fluor-phenyl)-ethyl]-cyclohexylamino}-1-hydroxy-ethyl)-5-hydroxy-4H-benzo[1,4]oxazin-3-on

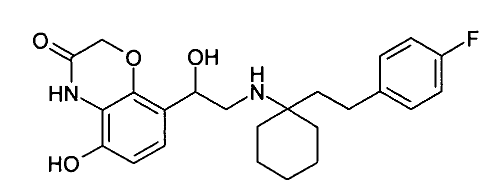

Hergestellt aus 560 mg (1.70 mmol) 5-Benzyloxy-8-(2,2-dihydroxy-acetyl)-4H-benzo[1,4]oxazin-3-on und 438 mg (1.70 mmol) 1-(2-(4-Fluor-phenyl)-ethyl]-cyclohexylamin Hydrochlorid nach der allgemeinen Arbeitsvorschrift 6. Ausbeute: 554 mg (70%, Hydrochlorid). Massenspektroskopie: [M+H]⁺ = 429.

### Beispiel 59: N-{2-Hydroxy-5-[1-hydroxy-2-(1-phenethyl-cyclohexylamino)-ethyl]-phenyl}-methanesulfonamid

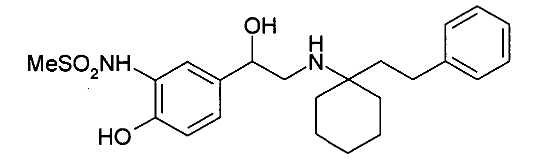

Hergestellt aus 645 mg (1.70 mmol) N-[2-Benzyloxy-5-(2-ethoxy-2-hydroxy-acetyl)-phenyl]-methanesulfonamid und 408 mg (1.70 mmol) 1-Phenethylcyclohexylamin Hydrochlorid nach der allgemeinen Arbeitsvorschrift 6. Ausbeute: 336 mg (42%, Hydrochlorid). Massenspektroskopie: [M+H]⁺ = 433.

### Beispiel 60: N-{5-[2-(1-Benzyl-cyclopentylamino)-1-hydroxy-ethyl]-2-hydroxy-phenyl}-methanesulfonamid

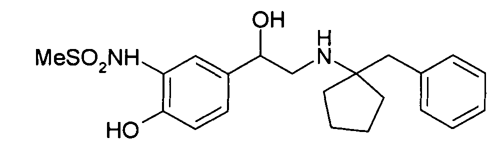

Hergestellt aus 645 mg (1.70 mmol) N-[2-Benzyloxy-5-(2-ethoxy-2-hydroxy-acetyl)-phenyl]-methanesulfonamid und 360 mg (1.70 mmol) 1-Benzylcyclopentylamin Hydrochlorid nach der allgemeinen Arbeitsvorschrift 6. Ausbeute: 346 mg (46%, Hydrochlorid). Massenspektroskopie: [M+H]⁺ = 405.

### Beispiel 61: N-(5-{2-[1-(4-Fluor-benzyl)-cyclopentylamino]-1-hydroxy-ethyl}-2-hydroxyphenyl)-methanesulfonamid

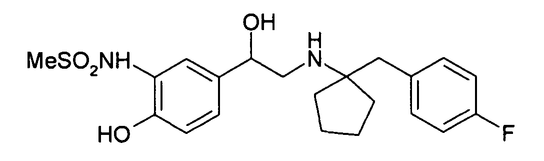

Hergestellt aus 645 mg (1.70 mmol) N-[2-Benzyloxy-5-(2-ethoxy-2-hydroxy-acetyl)-phenyl]-methanesulfonamid und 414 mg (1.70 mmol) 1-(4-Fluor-benzyl)-cyclopentylamin Hydrochlorid nach der allgemeinen Arbeitsvorschrift 6. Ausbeute: 485 mg (62%, Hydrochlorid). Massenspektroskopie: [M+H]⁺ = 423.

### Beispiel 62: N-[2-Hydroxy-5-(1-hydroxy-2-{1-[2-(4-methoxy-phenyl)-ethyl]-cyclohexylamino}-ethyl)-phenyl]-methanesulfonamid

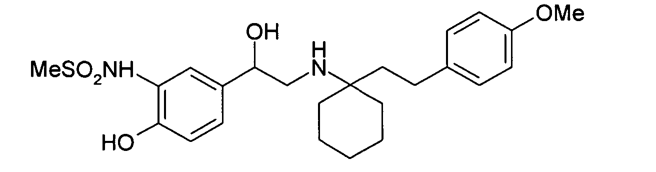

Erhalten aus 645 mg (1.70 mmol) N-[2-Benzyloxy-5-(2-ethoxy-2-hydroxy-acetyl)-phenyl]-methanesulfonamid und 458 mg (1.70 mmol) 1-[2-(4-Methoxy-phenyl)-ethyl]-cyclohexylamin Hydrochlorid nach der allgemeinen Arbeitsvorschrift 6. Ausbeute: 544 mg (64%, Hydrochlorid). Massenspektroskopie: [M+H]⁺ = 463.

### Beispiel 63: N-(2-Hydroxy-5-{1-hydroxy-2-[1-(4-methoxy-benzyl)-cyclopentylamino]-ethyl}-phenyl)-methanesulfonamid

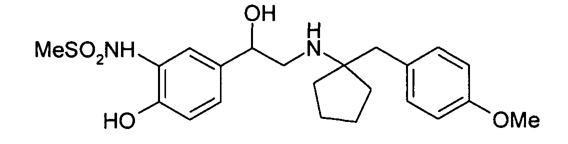

Hergestellt aus 645 mg (1.70 mmol) N-[2-benzyloxy-5-(2-ethoxy-2-hydroxy-acetyl)-phenyl]-methanesulfonamid und 411 mg (1.70 mmol) 1-(4-Methoxy-benzyl)-cyclopentylamin Hydrochlorid nach der allgemeinen Arbeitsvorschrift 6. Ausbeute: 369 mg (46%, Hydrochlorid). Massenspektroskopie: [M+H]⁺ = 435.

### Beispiel 64: N-(2-Hydroxy-5-{1-hydroxy-2-[1-(4-methoxy-benzyl)-cyclohexylamino]-ethyl}-phenyl)-methanesulfonamid

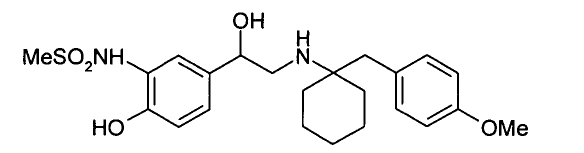

Hergestellt aus 645 mg (1.70 mmol) N-[2-Benzyloxy-5-(2-ethoxy-2-hydroxy-acetyl)-phenyl]-methanesulfonamid und 435 mg (1.70 mmol) 1-(4-Methoxy-benzyl)-cyclohexylamin Hydrochlorid nach der allgemeinen Arbeitsvorschrift 6. Ausbeute: 562 mg (68%, Hydrochlorid). Massenspektroskopie: [M+H]⁺ = 449.

### Beispiel 65: N-[5-(2-{1-[2-(4-Fluor-phenyl)-ethyl]-cyclohexylamino}-1-hydroxy-ethyl)-2-hydroxy-phenyl]-methanesulfonamid

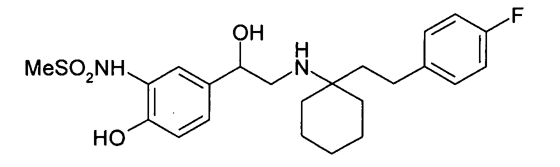

Hergestellt aus 645 mg (1.70 mmol) N-[2-Benzyloxy-5-(2-ethoxy-2-hydroxy-acetyl)-phenyl]-methanesulfonamid und 438 mg (1.70 mmol) 1-[2-(4-Fluor-phenyl)-ethyl]-cyclohexylamin Hydrochlorid nach der allgemeinen Arbeitsvorschrift 6. Ausbeute 614 mg (74%, Hydrochlorid). Massenspektroskopie: [M+H]⁺ = 451.

Wie gefunden wurde, zeichnen sich die Verbindungen der Formel **1** durch vielfältige Anwendungsmöglichkeiten auf therapeutischem Gebiet aus. Hervorzuheben sind solche Anwendungsmöglichkeiten, für welche die erfindungsgemäßen Verbindungen der Formel **1** aufgrund ihrer pharmazeutischen Wirksamkeit als Betamimetikum bevorzugt zur Anwendung gelangen können.

Dies sind beispielsweise die Behandlung von entzündlichen und obstruktiven Atemwegserkrankungen, die Hemmung verfrüht einsetzender Wehen in der Geburtshilfe (Tokolyse), die Wiederherstellung des Sinusrhythmus im Herzen bei atrioventrikulärem Block, sowie die Behebung bradykaler Herzrhythmusstörungen (Antiarrhythmikum), die Therapie des Kreislaufschocks (Gefäßerweiterung und Steigerung des Herzzeitvolumens) sowie die Behandlung von Juckreiz und Entzündungen der Haut.

Die erfindungsgemäßen Verbindungen sind insbesondere bei der Therapie von Atemwegserkrankungen einsetzbar. Ein bevorzugter Aspekt der vorliegenden Erfindung betrifft daher die Verwendung von Verbindungen der allgemeinen Formel **1** zur Herstellung eines Arzneimittels zur Behandlung von Erkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist die Verwendung von Verbindungen der allgemeinen Formel **1** zur Herstellung eines Arzneimittels zur Behandlung von Obstruktive Lungenerkrankungen die ausgewählt sind aus der Gruppe bestehend aus Asthma Bronchiale, pädiatrisches Asthma, schweres Asthma, akuter Asthma-Anfall, chronische Bronchitis und COPD (chronisch obstruktive pulmonale Erkrankung), wobei die Verwendung zur Herstellung eines Arzneimittels zur Behandlung von Asthma Bronchiale und COPD erfindungsgemäß besonders bevorzugt ist.

Bevorzugt ist ferner die Verwendung von Verbindungen der allgemeinen Formel **1** zur Herstellung eines Arzneimittels zur Behandlung von Lungenemphysemen die ihren Ursprung haben in COPD oder α1-Proteinase-Inhibitor-Mangel.

Bevorzugt ist ferner die Verwendung von Verbindungen der allgemeinen Formel **1** zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt ist ferner die Verwendung von Verbindungen der allgemeinen Formel **1** zur Herstellung eines Arzneimittels zur Behandlung von Interstitiellen Lungenerkrankungen, die ausgewählt sind aus der Gruppe bestehend aus infektiös bedingte Pneumonien, wie beispislweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, sytemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die Verwendung von Verbindungen der allgemeinen Formel **1** zur Herstellung eines Arzneimittels zur Behandlung von Zystischer Fibrose bzw. Mukoviszidose.

Bevorzugt ist ferner die Verwendung von Verbindungen der allgemeinen Formel **1** zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die Verwendung von Verbindungen der allgemeinen Formel **1** zur Herstellung eines Arzneimittels zur Behandlung von Bronchiektasen.

Bevorzugt ist ferner die Verwendung von Verbindungen der allgemeinen Formel **1** zur Herstellung eines Arzneimittels zur Behandlung von ARDS (adult respiratory distress syndrom).

Bevorzugt ist ferner die Verwendung von Verbindungen der allgemeinen Formel **1** zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorliegende Erfindung die Verwendung der Verbindungen der Formel **1** zur Herstellung eines Arzneimittels zur Behandlung von Asthma oder COPD. Von besonderer Bedeutung ist ferner die vorstehend genannte Verwendung von Verbindungen der Formel **1** zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Geeignete Anwendungsformen zur Applikation der Verbindungen der Formel **1** sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.
Säfte der erfindungsgemäßen Wirkstoffe können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilfslösungsmittel eingesetzt werden können, hergestellt und in Injektionsflaschen oder Ampullen oder Infusionsflaschen abgefüllt.

Die eine oder mehrere Wirkstoffe enthaltenden Kapseln können beispielsweise hergestellt werden, indem man die Wirkstoffe mit inerten Trägern, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide), synthetische Gesteinsmehle (z.B. hochdisperse Kieselsäure und Silikate), Zucker (z.B. Rohr-, Milch- und Traubenzucker), Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Bei der erfindungsgemäß bevorzugten Applikation der Verbindungen der Formel **1** zur Therapie von Asthma oder COPD werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

Erfindungsgemäß einsetzbare Inhalationspulver können **1** entweder allein oder im Gemisch mit geeigneten physiologisch unbedenkliche Hilfsstoffen enthalten.

Sind die Wirkstoffe **1** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfsstoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff **1**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt.

Die erfindungsgemäßen Inhalationspulver können mittels aus dem Stand der Technik bekannten Inhalatoren appliziert werden.

Erfindungsgemäße treibgashaltige Inhalationsaerosole können **1** im Treibgas gelöst oder in dispergierter Form enthalten. Hierbei können **1** in getrennten Darreichungsformen oder in einer gemeinsamen Darreichungsform enthalten sein, wobei **1** entweder beide gelöst, beide dispergiert oder jeweils nur eine Komponente gelöst und die andere dispergiert enthalten sein können.

Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie *n*-Propan, *n*-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannten Inhalatoren (MDIs = metered dose inhalers) appliziert werden.

Ferner kann die Applikation der erfindungsgemäßen Wirkstoffe **1** in Form von treibgasfreien Inhalationslösungen und Inhalationssuspensionen erfolgen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1** enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders bevorzugt unter 20 mg/ 100ml. Generell sind solche Inhalationslösungen bevorzugt, in denen der Gehalt an Natriumedetat bei 0 bis 10mg/100ml liegt.

Den treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.
Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.

Bevorzugte Formulierungen enthalten außer dem Lösungsmittel Wasser und dem Wirkstoff **1** nur noch Benzalkoniumchlorid und Natriumedetat.
In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Milligrammbereich liegen.

Ein weiterer Aspekt der vorliegenden Erfindung betrifft die vorstehend genannten pharmazeutische Formulierungen als solche, die durch einen Gehalt an einer Verbindung der Formel **1** gekennzeichnet sind, besonders bevorzugt die vorstehend genannten inhalativ applizierbaren pharmazeutischen Formulierungen.

Die nachfolgenden Formulierungsbeipiele illustrieren die vorliegende Erfindung ohne sie jedoch in ihrem Umfang zu beschränken:

| A) Ampullenlösung | | |
|---|---|---|
| | Wirkstoff der Formel **1** | 25 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| B) Dosieraerosol (Suspension) | | |
|---|---|---|
| | Wirkstoff der Formel **1** | 0.03 Gew.% |
| | Sorbitantrioleat | 0.6 Gew. % |
| | HFA134A:HFA227 2:1 | 99.37 Gew.% |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden

| C) Dosieraerosol (Lösung) | | |
|---|---|---|
| | Wirkstoff der Formel 1 | 0.03 Gew.% |
| | Ethanol abs. | 20 Gew.% |
| | wässrige HCl 0.01 mol/l | 2.0 Gew.% |
| | HFA134A | 77.97 Gew.% |

Die Herstellung der Lösung erfolgt in üblicher Art und Weise durch Mischen der einzelnen Bestandteile.

| D) Inhalationspulver | | |
|---|---|---|
| | Wirkstoff der Formel **1** | 80 µg |
| | Lactose Monohydrat ad | 10 mg |

Die Herstellung des Inhalationspulvers erfolgt in üblicher Art und Weise durch Mischen der einzelnen Bestandteile.

## Patentansprüche

1. Verbindungen der Formel **1** 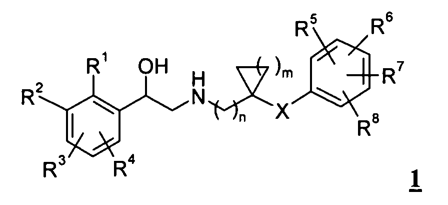 worin
n 0 oder 1;
m 1, 2, 3 oder 4;
X eine Einfachbindung oder eine der zweibindigen Gruppen C₂-C₆-Alkenylen, -O-C₁-C₆-Alkylen, -NH-C₁-C₆-Alkylen, -S-C₁-C₆-Alkylen oder C₁-C₆-Alkylen;
R¹ Wasserstoff;
R² -C₁-C₄-Hydroxyalkyl oder Halogen,
oder
R¹ und R² gemeinsam eine zweibindige Gruppe ausgewählt aus -O-CH₂-C(O)-NH-, -CH₂-CH₂-C(O)-NH-, -CH=CH-C(O)-NH-, -NH-CH₂-C(O)-NH-, -S-CH₂-C(O)-NH-, -O-C(O)-NH-, -S-C(O)-NH-, -NH-C(O)-NH-, und -O-CH₂-SO₂-NH-, in der jeweils ein oder 2 Wasserstoffatome ersetzt sein können durch einen oder zwei Reste ausgewählt aus der Gruppe bestehend aus -C₁-C₄-Alkyl, OH, oder Halogen;
R³ und R⁴ gleich oder verschieden, ein Rest ausgewählt aus der Gruppe bestehend aus Wasserstoff, OH, Halogen, -C₁-C₆-Alkyl,-C₁-C₆-Haloalkyl, -C₁-C₆-Hydroxyalkyl, NH₂, NH(-C₁-C₄-Alkyl) und N(-C₁-C₄-Alkyl)₂;
R⁵, R⁶, R⁷ und R⁸, gleich oder verschieden, Wasserstoff, OR⁹, -C₁-C₆-Alkyl, -C₁-C₆-Haloalkyl, -C₁-C₆-Hydroxyalkyl, -C₃-C₆-Cycloalkyl, -C₃-C₆-Hydroxycycloalkyl, -CN, NO₂, -COR⁹, -COOR⁹, -CONR¹⁰R¹¹, -NR¹⁰R¹¹, -NR¹⁰COR⁹, -NR¹⁰SO₂R¹², -SR¹², -SOR¹², -SO₂R¹², -SO₂NR¹⁰R¹¹ oder Halogen, oder
zwei der Reste R⁵, R⁶, R⁷ und R⁸ bilden, sofern sie am substituierenden Phenylring vicinal ständig sind, gemeinsam eine zweibindige Gruppe ausgewählt aus C₂-C₆-Alkylen, C₂-C₆-Alkenylen und -O-C₁-C₆-alkylen-O- in der jeweils ein oder 2 Wasserstoffatome ersetzt sein können durch einen oder zwei Reste ausgewählt aus der Gruppe bestehend aus -C₁-C₄-Alkyl, -C₁-C₄-Alkoxy, OH, oder Halogen;
R⁹ Wasserstoff, C₁-C₄-Alkyl, C₆-C₁₀-Aryl oder C₆-C₁₀-Aryl-C₁-C₄-alkylen;
R¹⁰ und R¹¹ gleich oder verschieden, Wasserstoff, C₁-C₄-Alkyl, C₆-C₁₀-Aryl oder C₆-C₁₀-Aryl-C₁-C₄-alkylen;
R¹² C₁-C₄-Alkyl, C₆-C₁₀-Aryl oder C₆-C₁₀-Aryl-C₁-C₄-alkylen,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

2. Verbindungen der allgemeinen Formel **1** nach Anspruch 1,
worin
n 0 oder 1;
m 1, 2, 3 oder 4;
X eine Einfachbindung oder eine der zweibindigen Gruppen C₂-C₄-Alkenylen, -O-C₁-C₄-Alkylen, -NH-C₁-C₄-Alkylen, -S-C₁-C₄-Alkylen oder C₁-C₄-Alkylen;
R¹ Wasserstoff;
R² -CH₂-CH₂-OH, -CH₂-OH, Fluor, Chlor oder Brom oder
R¹ und R² gemeinsam eine zweibindige Gruppe ausgewählt aus -O-CH₂-C(O)-NH-, -CH₂-CH₂-C(O)-NH-, -CH=CH-C(O)-NH-, -NH-CH₂-C(O)-NH-, -O-C(O)-NH-, -NH-C(O)-NH- und -O-CH₂-SO₂-NH-, in der jeweils ein oder 2 Wasserstoffatome ersetzt sein können durch einen oder zwei Reste ausgewählt aus der Gruppe bestehend aus Methyl, OH, Fluor, Chlor, oder Brom;
R³ und R⁴ gleich oder verschieden, ein Rest ausgewählt aus der Gruppe bestehend aus Wasserstoff, OH, Halogen, -C₁-C₄-Alkyl, -C₁-C₄-Haloalkyl, -C₁-C₄-Hydroxyalkyl, NH₂, NH(-C₁-C₄-Alkyl) und N(-C₁-C₄-Alkyl)₂;
R⁵, R⁶, R⁷ und R⁸, gleich oder verschieden, Wasserstoff, OR⁹, -C₁-C₄-Alkyl, -C₁-C₄-Haloalkyl, -C₁-C₄-Hydroxyalkyl, -C₃-C₆-Cycloalkyl, -C₃-C₆-Hydroxycycloalkyl, -CN, NO₂, -COR⁹, -COOR⁹, -CONR¹⁰R¹¹, -NR¹⁰R¹¹, -NR¹⁰COR⁹, -NR¹⁰SO₂R¹², -SR¹², -SOR¹², -SO₂R¹², -SO₂NR¹⁰R¹¹, Fluor, Chlor oder Brom, oder
zwei der Reste R⁵, R⁶, R⁷ und R⁸ bilden, sofern sie am substituierenden Phenylring vicinal ständig sind, gemeinsam eine zweibindige Gruppe ausgewählt aus C₂-C₄-Alkylen, C₂-C₄-Alkenylen und -O-C₁-C₄-Alkylen-O- in der jeweils ein oder 2 Wasserstoffatome ersetzt sein können durch einen oder zwei Reste ausgewählt aus der Gruppe bestehend aus Methyl, Methoxy, OH, Fluor, Chlor oder Brom;
R⁹ Wasserstoff, Methyl, Ethyl, Phenyl, Naphthyl, Benzyl, Naphthylmethyl oder 2- Phenylethyl;
R¹⁰ und R¹¹ gleich oder verschieden, Wasserstoff, Methyl, Ethyl, Phenyl, Naphthyl, Naphthylmethyl, Benzyl oder 2-Phenylethyl;
R¹² Methyl, Ethyl, Phenyl, Naphthyl, Naphthylmethyl, Benzyl oder 2-Phenylethyl,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

3. Verbindungen der allgemeinen Formel **1** nach Anspruch 1 oder 2,
worin
n 0 oder 1;
m 1, 2, 3 oder 4;
X eine Einfachbindung oder eine der zweibindigen Gruppen -CH₂-, -CH₂-CH₂-, -CH₂-CH₂-CH₂-, -CH=CH-, -CH₂-CH=CH-, -CH₂-O-, -CH₂-CH₂-O-, -CH₂-NH₂-, -CH₂-CH₂-NH₂-;
R¹ Wasserstoff;
R² -CH₂-CH₂-OH, -CH₂-OH, Fluor, Chlor oder Brom oder
R¹ und R² gemeinsam eine zweibindige Gruppe ausgewählt aus -O-CH₂-C(O)-NH-, -CH₂-CH₂-C(O)-NH-, -CH=CH-C(O)-NH-, oder -O-C(O)-NH-, in der jeweils ein oder 2 Wasserstoffatome ersetzt sein können durch einen oder zwei Reste ausgewählt aus der Gruppe bestehend aus Methyl, OH, Fluor, Chlor, oder Brom, bevorzugt Methyl;
R³ und R⁴ gleich oder verschieden, ein Rest ausgewählt aus der Gruppe bestehend aus Wasserstoff, OH, Fluor, Chlor, Brom, Methyl, Ethyl, -CH₂F, -CHF₂, -CF₃, -CH₂-CH₂-OH, -CH₂-OH, NH₂, NH(Methyl) und N(Methyl)₂;
R⁵, R⁶, R⁷ und R⁸, gleich oder verschieden, Wasserstoff, OH, Methyl, Ethyl, Propyl, Butyl, -CH₂F, -CHF₂, -CF₃, -CH₂-CH₂F, -CH₂-CHF₂, -CH₂-CF₃, -CH₂-CH₂-OH, -CH₂-OH, Cyclpropyl, Cyclobutyl, Cyclopentyl, HO-Cyclpropyl, HO-Cyclobutyl, HO-Cyclopentyl, -CN, NO₂, -COPhenyl, -COOMethyl, -COOEthyl, -CONH₂, -CONHMethyl, -CONHPhenyl, -CONHBenzyl, -CON(Methyl)₂, NH₂, NH(Methyl), N(Methyl)₂, -NHCOMethyl, -NHCOPhenyl, -NHSO₂Methyl, -NHSO₂Phenyl, -NHSO₂-Phenyl-CH₃, -SO₂Methyl, -SO₂-Phenyl, -SO₂-Phenyl-CH₃, -SO₂NH₂, Fluor, Chlor oder Brom, oder
zwei der Reste R⁵, R⁶, R⁷ und R⁸ bilden, sofern sie am substituierenden Phenylring vicinal ständig sind, gemeinsam eine zweibindige Gruppe ausgewählt aus -CH₂-CH₂-CH₂-, -CH₂-CH₂-CH₂-CH₂-, -CH₂-CH=CH-,
-CH=CH-CH=CH-, -O-CH₂-O- und -O-CH₂-CH₂-O- in der jeweils ein oder 2 Wasserstoffatome ersetzt sein können durch einen oder zwei Reste ausgewählt aus der Gruppe bestehend aus Methyl, Methoxy, OH oder Fluor,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

4. Verbindungen der allgemeinen Formel **1** nach einem der Ansprüche 1, 2 oder 3,
worin
X eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-, bedeutet
und worin die Gruppen R¹, R², R³, R⁴, R⁵, R⁶, R⁷, R⁸, n und m die in den Ansprüchen 1 bis 3 genannten Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

5. Verbindungen der allgemeinen Formel **1** nach einem der Ansprüche 1, 2 oder 3,
worin
R¹ Wasserstoff;
R² Methyl, Ethyl, CHF₂, CH₂F, CF₃, -CH₂-CH₂-OH, -CH₂-OH, Fluor, Chlor oder Brom, oder
R¹ und R² gemeinsam eine zweibindige Gruppe ausgewählt aus -O-CH₂-C(O)-NH-, -CH₂-CH₂-C(O)-NH-, -CH=CH-C(O)-NH- und -CH=CH-CH=CH-, bedeuten,
und worin die Gruppen R³, R⁴, R⁵, R⁶, R⁷, R⁸, n, m und X die in den Ansprüchen 1 bis 3 genannten Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

6. Verbindungen der allgemeinen Formel **1** nach einem der Ansprüche 1, 2 oder 3,
worin
R³ und R⁴ gleich oder verschieden, ein Rest ausgewählt aus der Gruppe bestehend aus Wasserstoff, OH, Fluor, Chlor, Brom, Methyl, Ethyl, CHF₂, CH₂F, CF₃, -CH₂-CH₂-OH, -CH₂-OH, NH₂, NHMethyl, NHEthyl, N(Methyl)₂ und N(Ethyl)₂, bevorzugt ausgewählt aus der Gruppe bestehend aus Wasserstoff, OH, Fluor, CH₂F, CF₃ und NH₂, bedeuten,
und worin die Gruppen R¹, R², R⁵, R⁶, R⁷, R⁸, n, m und X die in den Ansprüchen 1 bis 3 genannten Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

7. Verbindungen der allgemeinen Formel **1** nach einem der Ansprüche 1, 2 oder 3,
worin
R⁵, R⁶, R⁷ und R⁸, gleich oder verschieden, Wasserstoff, OH, Methyl, Ethyl, Propyl, Butyl, CHF₂, CH₂F, CF₃, -CH₂-CH₂-OH, -CH₂-OH, Methyloxy, Ethyloxy, Propyloxy, Butyloxy, Cyclopropyl, Hydroxycyclopropyl, NH₂, NHMethyl, N(Methyl)₂, Fluor, Chlor oder Brom, oder
zwei der Reste R⁵, R⁶, R⁷ und R⁸ gemeinsam, sofern sie am substituierenden Phenylring vicinal ständig sind, die zweibindige Gruppe
-CH=CH-CH=CH- in der jeweils ein oder 2 Wasserstoffatome ersetzt sein können durch einen oder zwei Reste ausgewählt aus der Gruppe bestehend aus Methyl, Methoxy, OH, Fluor, Chlor oder Brom, bedeuten,
und worin die Gruppen R¹, R², R³, R⁴ n, m und X die in den Ansprüchen 1 bis 3 genannten Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

8. Verbindungen der allgemeinen Formel **1** nach einem der Ansprüche 1, 2, 3 oder 8,
worin
R⁵, R⁶, R⁷ und R⁸, gleich oder verschieden, Wasserstoff, OH, Methyl, Ethyl, Propyl, Butyl, CHF₂, CH₂F, CF₃, Methyloxy, Ethyloxy, Propyloxy, Butyloxy, Cyclopropyl, Hydroxycyclopropyl, NH₂, Fluor, Chlor oder Brom,
bedeuten und worin die Gruppen R¹, R², R³, R⁴ n, m und X die in den Ansprüchen 1 bis 3 genannten Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

9. Verbindungen der allgemeinen Formel **1** nach einem der Ansprüche 1 bis 8, **dadurch gekennzeichnet, dass** es sich um Verbindungen der Formel **1a** handelt, 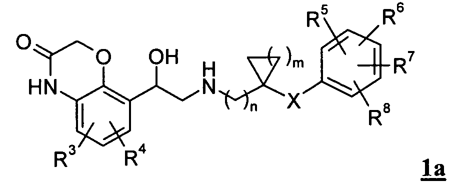 worin
n 0 oder 1, bevorzugt 0;
m 1, 2, 3 oder 4, bevorzugt 1;
X eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-,
R³ Wasserstoff;
R⁴ OH;
R⁵, R⁶, R⁷ und R⁸, gleich oder verschieden, Wasserstoff, OR⁹, -C₁-C₄-Alkyl, -C₁-C₄-Haloalkyl, -C₁-C₄-Hydroxyalkyl, -C₃-C₆-Cycloalkyl, -C₃-C₆-Hydroxycycloalkyl, -CN, NO₂, -COR⁹, -COOR⁹, -CONR¹⁰R¹¹, -NR¹⁰R¹¹, -NR¹⁰COR⁹ -NR¹⁰SO₂R¹², -SR¹², -SOR¹², -SO₂R¹², -SO₂NR¹⁰R¹¹, Fluor, Chlor oder Brom, oder
zwei der Reste R⁵, R⁶, R⁷und R⁸ bilden, sofern sie am substituierenden Phenylring vicinal ständig sind, gemeinsam eine zweibindige Gruppe ausgewählt aus C₂-C₄-Alkylen, C₂-C₄-Alkenylen und -O-C₁-C₄-Alkylen-O- in der jeweils ein oder 2 Wasserstoffatome ersetzt sein können durch einen oder zwei Reste ausgewählt aus der Gruppe bestehend aus Methyl, Methoxy, OH, Fluor, Chlor oder Brom;
R⁹ Wasserstoff, Methyl, Ethyl, Phenyl, Naphthyl, Benzyl, Naphthylmethyl oder 2- Phenylethyl;
R¹⁰ und R¹¹ gleich oder verschieden, Wasserstoff, Methyl, Ethyl, Phenyl, Naphthyl, Naphthylmethyl, Benzyl oder 2-Phenylethyl;
R¹² Methyl, Ethyl, Phenyl, Naphthyl, Naphthylmethyl, Benzyl oder 2-Phenylethyl,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

10. Verbindungen der allgemeinen Formel **1a** nach Anspruch 9,
worin
n 0 oder 1, bevorzugt 0;
m 1, 2, 3 oder 4, bevorzugt 1;
X eine Einfachbindung oder eine der zweibindigen Gruppen -CH₂-, -CH₂-CH₂-, -CH₂-CH₂-CH₂-, -CH=CH-, -CH₂-CH=CH-, -CH₂-O-, -CH₂-CH₂-O-, -CH₂-NH₂-, -CH₂-CH₂-NH₂--, bevorzugt eine Einfachbindung oder eine der zweibindigen Gruppen -O-CH₂- und -CH₂-, besonders bevorzugt -CH₂-;
R³ Wasserstoff;
R⁴ Wasserstoff, Fluor, Methyl, OH oder CF₃, bevorzugt OH;
R⁵ und R⁸ gleich oder verschieden, Wasserstoff, Methyl, Methyloxy oder Fluor;
R⁶ und R⁷ gleich oder verschieden, Wasserstoff, OH, Methyl, Ethyl, CF₃, Methyloxy, Ethyloxy, Propyloxy, Butyloxy, Cyclopropyl, Hydroxycyclopropyl, NH₂ oder Fluor,
oder
R⁶ und R⁷, sofern sie am substituierenden Phenylring vicinal ständig sind, gemeinsam die zweibindige Gruppe -CH=CH-CH=CH- in der jeweils ein oder 2 Wasserstoffatome ersetzt sein können durch einen oder zwei Reste ausgewählt aus der Gruppe bestehend aus Methyl, Methoxy, OH, Fluor, Chlor und Brom,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

11. Verbindungen der allgemeinen Formel **1** nach einem der Ansprüche 1 bis 8, **dadurch gekennzeichnet, dass** es sich um Verbindungen der Formel **1b** handelt, 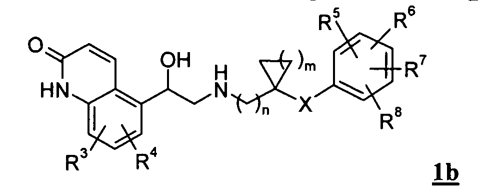 worin
n 0 oder 1, bevorzugt 0;
m 1, 2, 3 oder 4, bevorzugt 1;
X eine Einfachbindung oder eine der zweibindigen Gruppen -CH₂-, -CH₂-CH₂-, -CH₂-CH₂-CH₂-, -CH=CH-, -CH₂-CH=CH-, -CH₂-O-, -CH₂-CH₂-O-, -CH₂-NH₂-, -CH₂-CH₂-NH₂-, bevorzugt eine Einfachbindung oder eine der zweibindigen Gruppen -O-CH₂- und -CH₂-, besonders bevorzugt -CH₂-;
R³ Wasserstoff;
R⁴ Wasserstoff, Fluor, Methyl, OH oder CF₃, bevorzugt OH;
R⁵ und R⁸ gleich oder verschieden, Wasserstoff, Methyl, Methyloxy oder Fluor;
R⁶ und R⁷ gleich oder verschieden, Wasserstoff, OH, Methyl, Ethyl, CF₃, Methyloxy, Ethyloxy, Propyloxy, Butyloxy, Cyclopropyl, Hydroxycyclopropyl, NH₂ oder Fluor,
oder
R⁶ und R⁷, sofern sie am substituierenden Phenylring vicinal ständig sind, gemeinsam die zweibindige Gruppe -CH=CH-CH=CH- in der jeweils ein oder 2 Wasserstoffatome ersetzt sein können durch einen oder zwei Reste ausgewählt aus der Gruppe bestehend aus Methyl, Methoxy, OH, Fluor, Chlor und Brom,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

12. Verbindungen der allgemeinen Formel **1b** nach Anspruch 11,
worin
n 0 oder 1, bevorzugt 0;
m 1, 2, 3 oder 4, bevorzugt 1;
X eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-,
R³ Wasserstoff;
R⁴ OH;
R⁵ und R⁸ gleich oder verschieden, Wasserstoff, Methyl, Methyloxy oder Fluor;
R⁶ und R⁷ gleich oder verschieden, Wasserstoff, OH, Methyl, CF₃, Methyloxy, Ethyloxy, Propyloxy, Butyloxy, Hydroxycyclopropyl, NH₂ oder Fluor,
oder
R⁶ und R⁷, sofern sie am substituierenden Phenylring vicinal ständig sind, gemeinsam die zweibindige Gruppe -CH=CH-CH=CH-,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

13. Verbindungen der allgemeinen Formel **1b** nach Anspruch 11 oder 12,
worin
n 0 oder 1, bevorzugt 0;
m 1, 2, 3 oder 4, bevorzugt 1;
X die zweibindige Gruppe -CH₂-;
R³ Wasserstoff;
R⁴ OH;
R⁵, R⁶ und R⁸ gleich oder verschieden, Wasserstoff oder Methyl, bevorzugt Wasserstoff;
R⁷ Wasserstoff, OH, Methyl, CF₃, Methyloxy oder Ethyloxy, bevorzugt OH, CF₃ oder Methyloxy,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

14. Verbindungen der allgemeinen Formel **1** nach einem der Ansprüche 1 bis 8, **dadurch gekennzeichnet, dass** es sich um Verbindungen der Formel **1c** handelt, 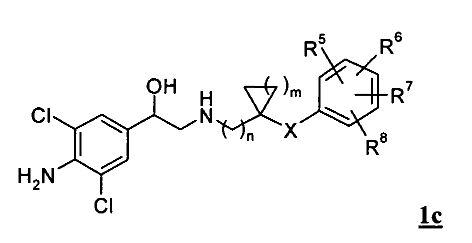 worin
n 0 oder 1, bevorzugt 0;
m 1, 2, 3 oder 4, bevorzugt 1;
X eine Einfachbindung oder eine der zweibindigen Gruppen -CH₂-, -CH₂-CH₂-, -CH₂-CH₂-CH₂-, -CH=CH-, -CH₂-CH=CH-, -CH₂-O-, -CH₂-CH₂-O-, -CH₂-NH₂-, -CH₂-CH₂-NH₂-;
R⁵ und R⁸ gleich oder verschieden, Wasserstoff, Methyl, Methyloxy oder Fluor;
R⁶ und R⁷ gleich oder verschieden, Wasserstoff, OH, Methyl, Ethyl, CF₃, Methyloxy, Ethyloxy, Propyloxy, Butyloxy, Cyclopropyl, Hydroxycyclopropyl, NH₂ oder Fluor,
oder
R⁶ und R⁷, sofern sie am substituierenden Phenylring vicinal ständig sind, gemeinsam die zweibindige Gruppe -CH=CH-CH=CH- in der jeweils ein oder 2 Wasserstoffatome ersetzt sein können durch einen oder zwei Reste ausgewählt aus der Gruppe bestehend aus Methyl, Methoxy, OH, Fluor, Chlor und Brom,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

15. Verbindungen der allgemeinen Formel **1c** nach Anspruch 14,
worin
n 0 oder 1, bevorzugt 0;
m 1, 2, 3 oder 4, bevorzugt 1;
X eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-,
R⁵ und R⁸ Wasserstoff;
R⁶ und R⁷ gleich oder verschieden, Wasserstoff, OH, Methyl, CF₃, Methyloxy oder Ethyloxy,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

16. Verbindungen der allgemeinen Formel **1c** nach Anspruch 14 oder 15,
worin
n 0 oder 1, bevorzugt 0;
m 1, 2, 3 oder 4, bevorzugt 1;
X die zweibindige Gruppe -CH₂-;
R⁵, R⁶ und R⁸ gleich oder verschieden, Wasserstoff oder Methyl, bevorzugt Wasserstoff;
R⁷ Wasserstoff, OH, Methyloxy oder Ethyloxy,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

17. Verbindungen der allgemeinen Formel **1** nach einem der Ansprüche 1 bis 8, **dadurch gekennzeichnet, dass** es sich um Verbindungen der Formel **1d** handelt, 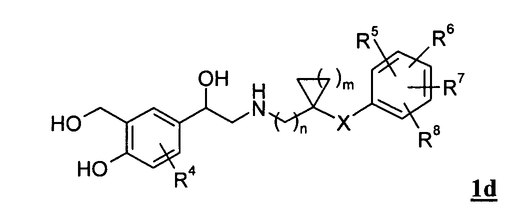 worin
n 0 oder 1, bevorzugt 0;
m 1, 2, 3 oder 4, bevorzugt 1;
X eine Einfachbindung oder eine der zweibindigen Gruppen -CH₂-, -CH₂-CH₂-, -CH₂-CH₂-CH₂-, -CH=CH-, -CH₂-CH=CH-, -CH₂-O-, -CH₂-CH₂-O-, -CH₂-NH₂-, -CH₂-CH₂-NH₂-;
R⁴ Wasserstoff, Fluor, Methyl, OH oder CF₃, bevorzugt Wasserstoff;
R⁵ und R⁸ gleich oder verschieden, Wasserstoff, Methyl, Methyloxy oder Fluor;
R⁶ und R⁷ gleich oder verschieden, Wasserstoff, OH, Methyl, Ethyl, CF₃, Methyloxy, Ethyloxy, Propyloxy, Butyloxy, Cyclopropyl, Hydroxycyclopropyl, NH₂ oder Fluor,
oder
R⁶ und R⁷, sofern sie am substituierenden Phenylring vicinal ständig sind, gemeinsam die zweibindige Gruppe -CH=CH-CH=CH- in der jeweils ein oder 2 Wasserstoffatome ersetzt sein können durch einen oder zwei Reste ausgewählt aus der Gruppe bestehend aus Methyl, Methoxy, OH, Fluor, Chlor und Brom,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

18. Verbindungen der allgemeinen Formel **1d** nach Anspruch 17,
worin
n 0 oder 1, bevorzugt 0;
m 1, 2, 3 oder 4, bevorzugt 1;
X eine Einfachbindung oder eine der zweibindigen Gruppen -CH₂-, -CH₂CH₂-, -CH₂-O-, bevorzugt eine Einfachbindung oder eine der zweibindigen Gruppen -O-CH₂- und -CH₂-, besonders bevorzugt -CH₂-,
R⁴ Wasserstoff;
R⁵ und R⁸ gleich oder verschieden, Wasserstoff, Methyl, Methyloxy oder Fluor, bevorzugt Wasserstoff;
R⁶ undR⁷ gleich oder verschieden, Wasserstoff, OH, Methyl, CF₃, Methyloxy, Ethyloxy, Propyloxy, Butyloxy, NH₂ oder Fluor,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

19. Verbindungen der allgemeinen Formel **1d** nach Anspruch 17 oder 18,
worin
n 0 oder 1, bevorzugt 0;
m 1, 2, 3 oder 4, bevorzugt 1;
X die zweibindige Gruppe -CH₂-;
R⁴ Wasserstoff;
R⁵, R⁶ und R⁸ gleich oder verschieden, Wasserstoff oder Methyl, bevorzugt Wasserstoff;
R⁷ Wasserstoff, OH, Methyl, CF₃, Methyloxy oder Ethyloxy, bevorzugt OH, CF₃ oder Methyloxy,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, sowie gegebenenfalls in Form ihrer Solvate oder Hydrate.

20. Verbindungen der allgemeinen Formel **1** nach einem der Ansprüche 1 bis 19, **dadurch gekennzeichnet, dass** es sich um Enantiomere der Formel ***R*****-****1** handelt, 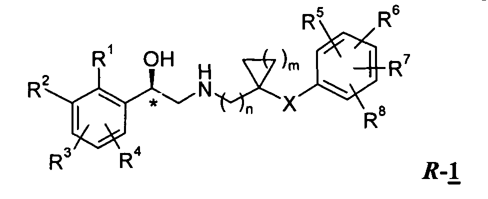 worin die Gruppen R¹, R², R³, R⁴, R⁵, R⁶, R⁷, R⁸, n, m und X die in den Ansprüchen 1 bis 19 genannten Bedeutungen haben können.

21. Verbindungen der Formel **1** gemäß einem der Ansprüche 1 bis 20 als Arzneimittel.

22. Verwendung der Verbindungen der Formel **1** gemäß einem der Ansprüche 1 bis 20 zur Herstellung eines Arzneimittels zur Behandlung von Erkrankungen, in denen therapeutisch wirksame Dosen eines Betamimetikums einen therapeutischen Nutzen entfalten können.

23. Verwendung nach Anspruch 22, **dadurch gekennzeichnet, dass** es sich um eine Erkrankung handelt, die ausgewählt ist aus der Gruppe bestehend aus Atemwegserkrankungen, Hemmung verfrüht einsetzender Wehen in der Geburtshilfe (Tokolyse), Wiederherstellung des Sinusrhythmus im Herzen bei atrioventrikulärem Block, Behebung bradykaler Herzrhythmusstörungen (Antiarrhythmikum), Kreislaufschock (Gefäßerweiterung und Steigerung des Herzzeitvolumens) und Juckreiz und Entzündungen der Haut.

24. Verwendung nach Anspruch 22 oder 23, **dadurch gekennzeichnet, dass** es sich um eine Atemwegserkrankung handelt, die ausgewählt ist aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

25. Verwendung nach Anspruch 24, **dadurch gekennzeichnet, dass** es sich um eine Obstruktive Lungenerkrankung handelt, die ausgewählt sind aus der Gruppe bestehend aus Asthma Bronchiale, pädiatrisches Asthma, schweres Asthma, akuter Asthma-Anfall, chronische Bronchitis und COPD (chronisch obstruktive pulmonale Erkrankung), wobei die Verwendung zur Herstellung eines Arzneimittels zur Behandlung von Asthma Bronchiale und COPD erfindungsgemäß besonders bevorzugt ist.

26. Verwendung nach Anspruch 24, **dadurch gekennzeichnet, dass** es sich um Lungenemphyseme handelt, die ihren Ursprung haben in COPD oder α1-Proteinase-Inhibitor-Mangel.

27. Verwendung nach Anspruch 24, **dadurch gekennzeichnet, dass** es sich um eine Restriktive Lungenerkrankung handelt, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, brochoalveoläres Karzinom und Lymphome.

28. Verwendung nach Anspruch 24, **dadurch gekennzeichnet, dass** es sich um eine Interstitielle Lungenerkrankung handelt, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, sytemische Sklerodermie oder Sarkoidose, Granulomatosen, wie beispielsweise Morbus Boeck, idiopathische interstitielle Pneumonie oder idiopathische pulmonäre Fibrose (IPF).

29. Verwendung nach Anspruch 24, **dadurch gekennzeichnet, dass** es sich bei der Erkrankung um Zystische Fibrose bzw. Mukoviszidose handelt.

30. Verwendung nach Anspruch 24, **dadurch gekennzeichnet, dass** es sich um Bronchitiden handelt, die ausgewählt sind aus der Gruppe bestehend aus Bronchitis aufgrund bakterieller oder viraler Infektion, Allergische Bronchitis und Toxische Bronchitis.

31. Verwendung nach Anspruch 24, **dadurch gekennzeichnet, dass** es sich bei der Erkrankung um Bronchiektasen oder ARDS (adult respiratory distress syndrom) handelt.

32. Verwendung nach Anspruch 24, **dadurch gekennzeichnet, dass** es sich bei den Lungenödemen um toxischer Lungenödeme nach Aspiration oder Inhalation von toxischen Substanzen und Fremdstoffen handelt.

33. Pharmazeutische Formulierungen **gekennzeichnet durch** den Gehalt an einer oder mehreren Verbindungen der Formel **1** gemäß einem der Ansprüche 1 bis 20.

34. Inhalativ applizierbare pharmazeutische Formulierung, **gekennzeichnet durch** einen Gehalt einer Verbindung der Formel **1** gemäß einem Ansprüche 1 bis 20.

35. Cycloalkylamine der Formel **2**, 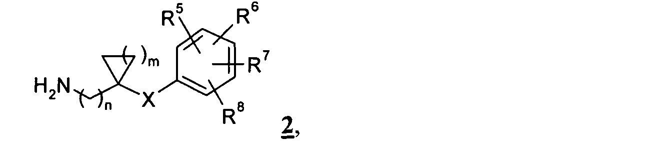 worin
n 0 oder 1;
m 1;
X eine Einfachbindung oder eine der zweibindigen Gruppen -O-, -NH, -S-, C₂-C₆- Alkenylen, -O-C₁-C₆-Alkylen, -NH-C₁-C₆-Alkylen, -S-C₁-C₆-Alkylen oder C₁-C₆- Alkylen;
R⁵, R⁶, R⁷ und R⁸, gleich oder verschieden, Wasserstoff, OR⁹, -C₁-C₆-Alkyl, -C₁-C₆-Haloalkyl, -C₁-C₆-Hydroxyalkyl, -C₃-C₆-Cycloalkyl, -C₃-C₆-Hydroxycycloalkyl, -CN, NO₂, -COR⁹, -COOR⁹, -CONR¹⁰R¹¹, -NR¹⁰R¹¹, -NR¹⁰COR⁹, -NR¹⁰SO₂R¹², -SR¹², -SOR¹², -SO₂R¹², -SO₂NR¹⁰R¹¹ oder Halogen,
oder
zwei der Reste R⁵, R⁶, R⁷ und R⁸ bilden, sofern sie am substituierenden Phenylring vicinal ständig sind, gemeinsam eine zweibindige Gruppe ausgewählt aus C₂-C₆-Alkylen, C₂-C₆-Alkenylen und -O-C₁-C₆-alkylen-O- in der jeweils ein oder 2 Wasserstoffatome ersetzt sein können durch einen oder zwei Reste ausgewählt aus der Gruppe bestehend aus -C₁-C₄-Alkyl, -C₁-C₄-Alkoxy, OH, oder Halogen;
R⁹ Wasserstoff, C₁-C₄-Alkyl, C₆-C₁₀-Aryl oder C₆-C₁₀-Aryl-C₁-C₄-alkylen;
R¹⁰ und R¹¹ gleich oder verschieden, Wasserstoff, C₁-C₄-Alkyl, C₆-C₁₀-Aryl oder C₆-C₁₀-Aryl-C₁-C₄-alkylen;
R¹² C₁-C₄-Alkyl, C₆-C₁₀-Aryl oder C₆-C₁₀-Aryl-C₁-C₄-alkylen;
bedeuten, gegebenenfalls in Form der einzelnen optischen Isomeren, Mischungen der einzelnen Enantiomeren oder Racemate.
